# EUROPEAN PATENT SPECIFICATION

(11) **EP 0 846 119 B1**
(45) Date of publication and mention of the grant of the patent: **13.11.2002**
(21) Application number: 96926638.6
(22) Date of filing: 13.08.1996
(51) Int. Cl.: C07D 495/04, A61K 31/495

(54) **THIENOPYRIMIDINE DERIVATIVES, THEIR PRODUCTION AND USE AS ENDOTHELIN ANTAGONISTS**
THIENOPYRIMIDINDERIVATE ALS ENDOTHELIN ANTAGONISTEN
DERIVES DE THIENOPYRIMIDINE, LEUR FABRICATION ET LEURS APPLICATIONS COMME ANTAGONISTES DE L'ENDOTHELINE

(30) Priority: 17.08.1995 JP 20949895
(43) Date of publication of application: 10.06.1998
(73) Proprietor: Takeda Chemical Industries, Ltd., Osaka-shi, Osaka 541-0045 (JP)
(72) Inventor: FURUYA, Shuichi, Ibaraki 305 (JP); CHOH, Nobuo, Ibaraki 305 (JP); WATANABE, Toshifumi, Osaka 586 (JP)
(74) Representative: von Kreisler, Alek, Dipl.-Chem.
(86) International application number: JP9602290
(87) International publication number: WO97007119

(56) References cited:
- EP-A- 0 640 606
- WO-A-93/08799

## Description

### Technical Field

The present invention relates to thienopyrimidine derivatives and salts thereof. The present invention further relates to methods for manufacturing the thienopyrimidine derivatives and the salts thereof, and pharmaceutical compositions containing the thienopyrimidine derivatives.

### Background Art

The possibility has been suggested that, among adult diseases which are being encountered with increasing frequencies in these years, ischemia-associated diseases such as cerebral infarction, angina pectoris, myocardial infarction, renal failure and hepatic disorder are mediated by endothelin. Endothelin is a peptide of 21 amino acid residues as produced and released from endothelial cells and endothelin-1, endothelin-2 and endothelin-3 have so far been identified. Throughout this specification, these endothelin species are collectively referred to as "endothelin".

Endothelin reportedly is a substance having the most potent and lasting vasoconstrictive, pressor and heart muscle contractility-increasing actions of all the physiological substances and synthetic substances so far known. It is suspected that these actions of this particular peptide are manifested through the endothelin receptors suspected to exist in the vascular smooth muscle fascia and elsewhere. As the endothelin receptors, endothelin-A and endothelin-B receptors (both are collectively referred to as endothelin receptors) are already known.

Therefore, any compound having an affinity for the endothelin receptors and showing endothelin antagonizing activity is likely to be effective in the prevention and treatment of ischemia-associated diseases (for example, cerebral infarction, angina pectoris, myocardial infarction, renal failure, and hepatic disorder) and the development of a drug of this type has been awaited in earnest. As synthetic compounds having endothelin receptor antagonist activity, the compounds described typically in EP-A-510526, EP-A-526708, PCT•WO-9308799, and Journal of Medicinal Chemistry, 37, 1553-1557, 1994 are known.

Recently, it has been pointed out that a thienopyrimidine derivative has endothelin receptor antagonist activity (European Patent Publication No. 640,606).

During the study of thienopyrimidine compounds, the present inventors have found that a thienopyrimidine compound which has a carboxyl group and a group capable of forming an anion in the molecule has particularly potent endothelin receptor antagonist activity. The inventors did further research on the basis of the above finding and have completed the present invention.

### Disclosure of Invention

The present invention provides:
(1) A compound of the formula: 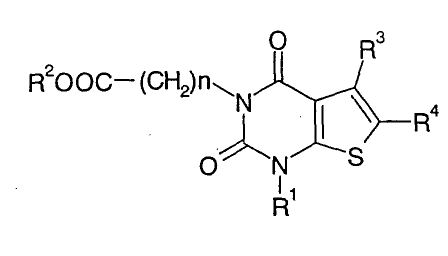 wherein each of R¹ and R² are (a) hydrogen, or (b) a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-membered nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxycarbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) a group of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido;
   R³ is a C₁₋₆ alkyl group substituted by a C₁₋₆ alkoxycarbonyl or a group of the formula: -NH-SO₂-R⁵ wherein R⁵ is (1) C₁₋₆ alkyl which may be substituted by halogen, or (2) C₆₋₁₄ aryl;
   R⁴ is a phenyl group which may be substituted by (1) C₁₋₄ alkoxy which may be substituted by C₁₋₆ alkoxy, carboxy, C₁₋₆ alkyl-carbamoyl, piperazinecarbonyl or halogen, (2) C₇₋₈ aralkyloxy, (3) C₁₋₄ alkyl which may be substituted by hydroxy, oxo or C₁₋₃ alkoxy, (4) C₁₋₆ alkanoyl, (5) C₂₋₄ alkenyloxy, (6) C₁₋₆ alkoxy-carbonyl or (7) C₁₋₆ alkyl-carbamoyl; and
   n is an integer of 1 to 3,
   or a salt thereof;
(2) a compound according to item (1) wherein R¹ is a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-memberd nitrogen containing heterocyclic group-carbonyl-or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) agroup of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido;
(3) a compound according to item 1, wherein R¹ is C₇₋₂₀ aralkyl group which may be substituted by halogen, nitro, hydroxy, cyano, C₁₋₄ alkyl, C₁₋₄ alkylthio or C₁₋₄ alkoxy;
(4) a compound according to item (1) wherein R¹ is benzyl which may be substituted by halogen or C₁₋₄ alkylthio;
(5) a compound according to item (1) wherein R² is a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-memberd nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) agroup of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy; C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido;
(6) a compound according to item (1) wherein R² is a C₁₋₁₀ alkyl group which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-memberd nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) a group of the formula: - NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido;
(7) a compound according to item (1) wherein R² is a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) hydroxy, (4) cyano, (5) C₁₋₄ alkylthio, (6) C₁₋₄ alkoxy, (7) C₁₋₆ alkyl-carbonyloxy or (8) C₃₋₆ cycloalkyl-oxycarbonyl;
(8) a compound according to item (1), wherein R² is hydrogen or a C₁₋₆ alkyl group which may be optionally substituted by C₁₋₆ alkyl-carbonyloxy or C₃₋₆ cycloalkyloxycarbonyl oxy;
(9) a compound according to item (1), wherein R³ is a C₁₋₆ alkyl group which is substituted by a C₁₋₆ alkoxycarbonyl group or a group of the formula: -NH-SO₂-R^{5'}, wherein R^{5'} is a C₁₋₆ alkyl group or a C₆₋₁₄ aryl group;
(10) a compound according to item (1), wherein R³ is a C₁₋₆ alkyl group which is substituted by a group of the formula: -NH-SO₂-R⁵, wherein R⁵ is (1) a C₁₋₆ alkyl group which may optionally be substituted by halogen or (2) a C₆₋₁₄ aryl group;
(11) a compound according to item (1), wherein R³ is a C₁₋₆ alkyl group which is substituted by a group of the formula: -NH-SO₂-R^{5'}, wherein R^{5'} is a C₁₋₆ alkyl group or a C₆₋₁₄ aryl group;
(12) a compound according to item (1), wherein R¹ is a benzyl group which may optionally be substituted by (1) halogen or (2) C₁₋₄ alkylthio,
   R² is a hydrogen atom or a C₁₋₄ alkyl group which may optionally be substituted by (1) C₁₋₆ alkyl-carbonyloxy or (2) C₃₋₆ cycloalkyl-oxycarbonyloxy,
   R³ is a C₁₋₆ alkyl group which is substituted by (1) a C₁₋₆ alkoxy-carbonyl group or (2) a group of the formula: -NH-SO₂-R^{5"} (wherein R^{5"} is (1) a C₁₋₃ alkyl group which may optionally be substituted by halogen or (2) a phenyl group,
   R⁴ is a phenyl group which is substituted by (1) C₁₋₄ alkoxy which may be substituted by C₁₋₆ alkoxy, carboxyl, C₁₋₆ alkyl-carbamoyl, piperazinecarbonyl or halogen, (2) C₇₋₈ aralkyloxy, (3) C₁₋₄ alkyl which may be substituted by C₁₋₃ alkoxy, (4) C₁₋₆ alkanoyl, (5) C₂₋₄ alkenyloxy, (6) C₁₋₆ alkoxy-carbonyl or (7) C₁₋₆ carbamoyl;
(13) 2,4(1H,3H)-dioxo-6-(4-methoxymethoxyphenyl)-1-(2-methylthiobenzyl)-5-(methanesulfonamidomethyl)-thieno[2,3-d]pyrimidine-3-acetic acid or its salt;
(14) 2,4(1H,3H)-dioxo-6-(4-methoxymethoxyphenyl)-1-(2-methylthiobenzyl)-5-(ethanesulfonamidomethyl)-thieno[2,3-d]pyrimidine-3-acetic acid or its salt;
(15) 2,4(1H,3H)-dioxo-6-(4-methoxyphenyl)-1-(2-methylthiobenzyl)-5-(methanesulfonamidomethyl)-thieno[2,3-d]pyrimidine-3-acetic acid or its salt;
(16) Ethyl 2,4(1H,3H)-dioxo-6-(4-methoxyphenyl)-1-(2-methylthiobenzyl)-5-(carboxymethyl)thieno[2,3-d]pyrimidine-3-acetate.
(17) A method for producing a compound as defined in item (1), which comprises subjecting a compound of the formula: 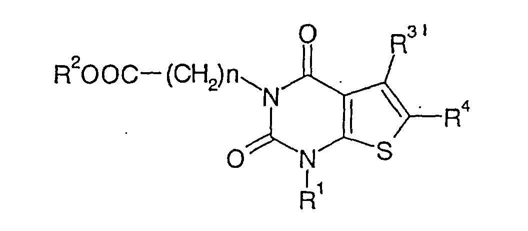 wherein R^{3'} is a C₁₋₆ alkyl group which is halogenated or cyanated, and other symbols have the same meaning as defined in item (1), to (1) a nucleophilic substitution reaction with a sulfonamide compound when R^{3'} is a C₁₋₆ alkyl group which is halogenated or (2) alkali-hydrolysis and the esterification when R^{3'} is a C₁₋₆ alkyl group which is cyanated;
(18) a pharmaceutical composition, which comprises a compound as defined in item (1) or (16) and a carrier, excipient or diluent therefor;
(19) a pharmaceutical composition according to item (18), which is a therapeutic drug for treating vasoconstriction in a mammal;
(20) a pharmaceutical composition according to item (19), wherein the vasoconstriction is in a coronary artery, coronary vein, cerebrovascular system or pulmonary vascular system;
(21) a pharmaceutical composition according to item (18), which is for antagonizing endothelin activity;
(22) a pharmaceutical composition according to item (21), which is a therapeutic drug for acute renal insufficiency, cardiac infarction or liver insufficiency;
(23) a pharmaceutical composition according to item (21), which is a treapeutic drug for hypofunction of an organ caused by a surgery or transplant;
(24) a pharmaceutical composition according to item (23), wherein the organ is liver;
(25) use of a compound as defined in item (1) or (16) for producing a pharmaceutical composition for the manufacture of a medicament for therapeutic application on vasoconstriction;
(26) use of a compound as defined in item (1) or (16) for producing a pharmaceutical composition for the manufacture of a medicament for therapeutic application on acute renal insufficiency, cardiac infarction or liver insufficiency.

The nucleus of the present compound, 2,4(1H,3H)-dioxo-thieno[2,3-d]pyrimidine, is shown below:

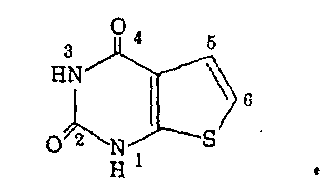

The group which is capable of forming an anion or a group convertible thereinto except carboxyl group includes tetrazolyl, an optionally substituted sulfonamide group, e.g. a group of the formula: -NH-SO₂-R⁵ wherein R⁵ is (1) a C₁₋₆ alkyl group which may optionally be substituted by halogen or (2) a C₆₋₁₄ aryl group, phosphono group and sulfo group, each of which may optionally be substituted by one or 2 of C₁₋₆ alkyl or acyl, e.g. C₂₋₅ alkanoyl, e.g. acetyl, propionyl, butyryl, valeryl, or C₆₋₁₄ arylcarbonyl, e.g. benzoyl.

The hydrocarbon residue in the optionally substituted hydrocarbon residue for the group R¹, the group R², the group R⁴ in the formula (I') and the group R⁶ mentioned below includes a hydrocarbon residue having one to 20 carbon atoms. As examples of the C₁₋₂₀ hydrocarbon residue, mention is made of C₁₋₁₀ alkyl, e.g. methyl, ethyl, propyl, isopropyl, n-butyl, isobutyl, t-butyl, pentyl, hexyl, heptyl, octyl, nonyl, decyl, etc, and among others, C₁₋₆ alkyl is preferable, C₃₋₁₀ cycloalkyl, e.g. cyclopropyl, cyclobutyl, cyclopentyl, cyclohexyl, cycloheptyl, cyclooctyl, cyclononyl, etc, and among others, C₃₋₆ cycloalkyl is preferable, C₇₋₁₀ bicycloalkyl, e.g. bicyclo[2,2,1]heptyl, bicyclo[2,2,2]octyl, bicyclo[3,2,1]octyl, bicyclo[3,2,1]nonyl, bicyclo[4,2,1]nonyl and bicyclo[4,3,1]decyl, etc, C₂₋₁₀ alkenyl, e.g. vinyl, allyl, isopropenyl, 1-butenyl, 2-butenyl, butadienyl, hexatrienyl, etc, and among others, C₂₋₆ alkenyl is preferable, C₆₋₁₄ aryl e.g. phenyl, naphthyl, anthryl, phenanthryl, acenaphthyl, anthracenyl, etc., among others, phenyl, 1-naphthyl, 2-naphthyl are preferable, and C₇₋₂₀ aralkyl, e.g. benzyl, phenethyl, benzhydryl, trityl, etc, and among others, C₇₋₈ aralkyl, e.g. benzyl and phenethyl are preferable.

The substituent which said hydrocarbon residue may optionally have includes but is not limited to (1) halogen, e.g. fluorine, chlorine, bromine, iodine, (2) nitro, (3) nitroso, (4) cyano, (5) hydroxyl group which may optionally be substituted by (i) C₁₋₆ alkyl, which may optionally be substituted by hydroxyl, C₁₋₆ alkoxy, C₁₋₃·alkoxy-C₁₋₃ alkoxy, C₁₋₃ alkylthio, oxy-C₁₋₃ alkoxy, carboxyl, carbamoyl, C₁₋₆ alkyl-carbamoyl, 5 to 7 membered nitrogen containing heterocyclic group-carbonyl or halogen, (ii) C₁₋₆ acyl, (iii) C₇₋₂₀ aralkyl, which may optionally be substituted by halogen, C₁₋₃ alkoxy or C₁₋₄ alkyl, (iv) C₆₋₁₄ aryl, which may optionally be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkyl-amino, (ix) C₁₋₃ alkoxy-carbonyl, (x) C₁₋₆ alkyl-carbonyl, (xi) C₃₋₆ cycloalkyloxycarbonyl or (xii) trifluorosulfonyl, (6) a group of the formula: -S(O)f-R⁶, wherein f is an integer of 0 to 2, R⁶ represents a hydrogen atom or a hydrocarbon residue which may optionally be substituted, the hydrocarbon residue has the same meaning as defined above, among others, C₁₋₆ alkyl, C₆₋₁₄ aryl, C₇₋₂₀ aralkyl are preferable, and as examples of the substituent to the hydrocarbon residue, mention is made of halogen, nitro, cyano, hydroxy, oxo, thioxo, carboxyl, cyano-C₆₋₁₄ aryl, halogeno-C₆₋₁₄ aryl, etc, (7) an optionally substituted amino group, which is represented by the formula: - NR⁹R¹⁰, wherein each of R⁹ and R¹⁰ are hydrogen, hydrocarbon residue, which has the same meaning as defined above, C₁₋₆ acyl or a 5 to 13 membered heterocyclic group which is mentioned below, (8) an optionally substituted carboxyl group of the formula: - CO-R¹¹ wherein R¹¹ denotes (i) hydrogen, (ii) hydroxyl, (iii) C₁₋₆ alkyl, (iv) C₁₋₆ alkoxy, (v) C₃₋₆ cycloalkyl, (vi) C₆₋₁₄ aryl, (vii) C₇₋₂₀ aralkyl, (viii) an optionally substituted amino group which is defined above or (vix) an optionally substituted 5- to 13-membered heterocyclic group which is mentioned below, (9) a 5-through 13-membered heterocyclic group containing 1-4 hetero-atom(s) selected from oxygen (O), sulfur (S) and nitrogen (N) as ring members, the heterocyclic group being optionally substituted by (i) halogen, (ii) C₁₋₄ alkyl, (iii) C₁₋₃ alkoxy, (iv) C₁₋₄ alkylthio, (v) phenoxy which may optionally be substituted by a halogen,
(10) sulfo, (11) C₆₋₁₄ aryl, e.g. phenyl, naphthyl, anthryl, phenanthryl, acenaphthyl, anthracenyl, etc, (12) C₃₋₇ cycloalkyl, (13) C₁₋₆ alkylenedioxy, e.g. methylenedioxy, ethylenedioxy, propylenedioxy, 2,2-dimethylenedioxy, etc, (14) oxo, (15) thioxo, (16) C₂₋₄ alkenyl, (17) C₃₋₄ alkynyl, e.g. propagyl, 2-butenyl, etc, (18) C₃₋₁₀ cycloalkyl, (19) C₂₋₁₀ alkenyl, e.g. vinyl, allyl, isopropenyl, 1-butenyl, 2-butenyl, butadienyl, hexatrienyl, etc., and among others, C₂₋₆ alkenyl is preferable, (20) C₇₋₂₀ aralkyl, which has the same meaning as defined above, (21) amidino, and
(22) azido.

When the hydrocarbon residue is cycloalkyl, cycloalkenyl, aryl or aralkyl, each of the group may have one to three of C₁₋₆ alkyl, e.g. methyl, ethyl, propyl, isopropyl, butyl, as a substituent. The C₁₋₆ alkyl group may further substituted by one to three of hydroxy, oxo, C₁₋₃ alkoxy, e.g. methoxy, ethoxy, n-propoxy, isopropoxy, C₁₋₃ alkylthio, halogen or carbamoyl.

The examples of the substituted alkyl, mention is made of (1) formyl, i.e. methyl is substituted by oxo, (2) carboxyl, i.e. methyl is substituted by oxo and hydroxy, (3) C₁₋₆ alkoxy-carbonyl, i.e. methyl is substituted by oxo and alkoxy, e.g. methoxycarbonyl, ethoxycarbonyl, t-butoxycarbonyl, hydroxy-C₁₋₆ alkyl, e.g. hydroxymethyl, hydroxyethyl, hydroxypropyl, hydroxybutyl, (4) C₁₋₃ alkoxy-C₁₋₆ alkyl, e.g. methoxymethyl, ethoxyethyl, ethoxybutyl, propoxymethyl, propoxyhexyl.

In the above optionally substituted hydrocarbon residue, the number of the substituent(s) is preferably 1 to 6, more preferably 1 to 5, and still preferably 1 to 3 and most preferably 1 to 2. The number of the substituent(s) which is substituted on the substituent is preferably 1 to 3, more preferably 1 or 2.

In the formula (I), n denotes 1 to 3, preferably 1 or 2, more preferably 1.

The C₁₋₆ alkyl group in the C₁₋₆ alkyl group which is substituted by a C₁₋₆ alkoxy-carbonyl group or a group of the formula: -NH-SO₂-R⁵ mentioned for R³ includes methyl, ethyl, n-propyl, isopropyl, n-butyl, isobutyl, sec-butyl, t-butyl, pentyl, hexyl, etc. Particularly preferred is methyl or ethyl.

The C₁₋₆ alkoxy in C₁₋₆ alkoxy-carbonyl group of R³ includes methoxy, ethoxy, propoxy, isopropoxy, n-butoxy, isobutoxy, t-butoxy, pentoxy, hexyloxy. In particular, C₁₋₄ alkoxy is preferable.

The C₁₋₆ alkyl group of the C₁₋₆ alkyl which may optionally be substituted by halogen of R⁵ includes the same groups as mentioned above. In particular, methyl or ethyl, is preferred.

The halogen includes fluorine, chlorine, bromine, iodine. Among others, fluorine and chloride is preferable.

The number of the substituent is preferably 1 to 3.

The C₆₋₁₄ aryl group of R⁵ includes phenyl, naphthyl, anthryl. Among others, phenyl is preferable.

The heterocyclic group of the optionally substituted heterocyclic group mentioned for R⁴ includes 3- through 13-membered, preferably 5- through 13-membered, heteroaromatic groups and non-aromatic saturated or unsaturated heterocyclic groups containing 1-4 hetero-atoms selected from among oxygen (O), sulfur (S) and nitrogen (N) as ring members.

The preferred heteroaromatic group includes monocyclic heteroaromatic groups such as furyl, thienyl, pyrrolyl, pyrrolinyl, oxazolyl, isoxazolyl, thiazolyl, isothiazolyl, imidazolyl, imidazolinyl, pyrazolyl, pyrazolinyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, triazinyl, 1,2,3-triazolyl, triazolidinyl, 1,2,4-triazolyl, tetrazolyl, pyridyl, pyridazinyl, pyrimidinyl, pyrazinyl, triazinyl, etc., and fused heteroaromatic groups such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenathridinyl, phenanthrolinyl, indolidinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.

The preferred nonaromatic heterocyclic group includes oxiranyl, azetidinyl, oxetanyl, thietanyl, thiazolidinyl, pyrrolidinyl, pyrazolidinyl, imidazolidinyl, tetrahydrofuryl, thioranyl, piperidyl, piperidinyl, tetrahydropyranyl, morpholinyl, thiomorpholinyl, piperazinyl, oxazolino, hexamethyleneamino, etc.

As the heterocyclic group, a 5 to 7 membered heretocyclic group is prefereble, and a 5 to 6 membered heterocyclic group is more prefereble.

The above heterocyclic groups may each have 1 or more, preferably 1-3, suitable substituents, which can be the same as the above-mentioned substituents for hydrocarbon residue.

The optionally substituted hydrocarbon residue of R¹ is preferably C₁₋₂₀ hydrocarbon residue. Among others, C₁₋₁₀ alkyl, C₃₋₁₀ cycloalkyl, C₂₋₁₀ alkenyl, C₆₋₁₄ aryl and C₇₋₂₀ aralkyl are preferable.

As R¹, an optionally substitued C₇₋₂₀ aralkyl is most preferable.

As preferable examples of the substituent in the optionally substituted hydrocarbon residue of R¹ is (1) halogen, (2) nitro, (3) cyano, (4) an optionally substituted hydroxyl group, (5) a group of the formula: -S(O)f-R⁶ wherein f denotes an integer of 0 to 2, and R⁶ is a hydrogen atom or an optionally substituted hydrocarbon residue, (6) an optionally substituted amino group or (7) an optionally substituted 5- or 6-membered heterocyclic group which contains 1 to 4 heteroatom(s) of oxygen, sulfur or nitrogen.

The group R¹ is preferably the group of the formula: -(CH₂)ₘQ, wherein m is an integer of 0 to 3 and Q is an optionally substituted C₆₋₁₄ aryl group, an optionally substituted C₃₋₁₀ cycloalkyl group or an optionally substituted 5 to 13-membered heterocyclic group.

As the above optionally substituted C₆₋₁₄ aryl group, a C₆₋₁₄ aryl group which may have one to three substituent(s) of halogen, nitro, cyano, amino, carboxyl which may be optionally substituted, C₁₋₆ alkylenedioxy, C₁₋₆ alkoxy, or C₁₋₆ alkylthio. The optionally substituted carboxyl has the same meaning of the above group of the formula: -CO-R¹¹.

In particular, Q is preferably C₆₋₁₄ aryl group optionally substituted by (1) halogen, (2) C₁₋₆ alkoxy, (3) C₁₋₆ alkylthio, (4) a group of the formula: -A-R¹² (wherein A and R¹² have the same meaning as defined above. Furthermore, Q is more preferably C₆₋₁₄ aryl which may be substituted by (1) halogen, (2) C₁₋₆ alkylthio or (3) C₁₋₆ alkoxy. As the aryl, phenyl is most preferable.

As the preferable group of R¹, mention is made of a C₇₋₂₀ aralkyl which is optionally substituted. As the preferable example of the substituent, mention is made of (1) halogen, (2) nitro, (3) hydroxy, (4) cyano, (5) C₁₋₄ alkyl, (6) C₁₋₄ alkylthio, (7) C₁₋₄ alkoxy. Among others, (1) halogen and (2) C₁₋₄ alkylthio is preferable, and C₁₋₄ alkylthio is most preferable. As the C₇₋₂₀ aralkyl, benzyl is most preferable.

The optionally substituted amino group a group of the formula: -NR⁹R¹⁰, wherein R⁹ and R¹⁰ are the same or different hydrogen, hydrocarbon residue, which has the same meaning as defined above, C₁₋₆ acyl or heterocyclic group which is mentioned below.

As the preferred group of R², mention is made of those of R¹.

Further, as the group R², hydrogen or an optionally substituted C₁₋₁₀ alkyl is preferable. As the alkyl, an optionally substituted C₁₋₆ alkyl is more preferable, and furthermore an optionally substituted C₁₋₄ alkyl is most preferable.

As the substituent on the alkyl of R², preferred examples are (1) halogen, (2) nitro, (3) cyano, (4) an optionally substituted hydroxyl group, (5) a group of the formula: -S(O)f-R⁶, wherein f denotes an integer of 0 to 2, and R⁶ is a hydrogen atom or an optionally substituted hydrocarbon residue, (6) an optionally substituted amino group or (7) an optionally substituted 5- or 6-membered heterocyclic group which contains 1 to 4 heteroatoms of oxygen, sulfur or nitrogen. Among others, as substituents, (1) halogen, (2) nitro, (3) hydroxy, (4) cyano, (5) C₁₋₄ alkylthio, (6) C₁₋₄ alkoxy, (7) C₁₋₆ alkyl-carbonyloxy, (8) C₃₋₆ cycloalkyl-oxycarbonyloxy are preferred. In these groups, (1) C₁₋₆ alkyl-carbonyloxy or (2) C₃₋₆ cycloalkyl-oxycarbonyloxy is most preferable.

As the group R³, preferable examples include (a) a C₁₋₆ alkyl group which is substituted by (1) a C₁₋₆ alkoxy-carbonyl group or (2) a group of the formula: -NH-SO₂-R^{5'}, wherein R^{5'} is a C₁₋₆ alkyl group or a C₆₋₁₄ aryl group, (b) a C₁₋₆ alkyl group which is substituted by a group of the formula: -NH-SO₂-R⁵, wherein R⁵ is (1) a C₁₋₆ alkyl group which may optionally substituted by halogen or (2) or C₆₋₁₄ aryl group, (c) a C₁₋₆ alkyl group which is substituted by a group of the formula: -NH-SO₂-R^{5'}, wherein R^{5'} is a C₁₋₆ alkyl group or a C₆₋₁₄ aryl group, (d) a C₁₋₆ alkyl group which is substituted by a group of the formula: -NH-SO₂-R^{5"}, wherein R^{5"} is a C₁₋₃ alkyl group which may optionally be substituted by halogen or a phenyl group, (e) a C₁₋₆ alkyl group which is substituted by a group of the formula: -NH-SO₂-R^{5'"}, wherein R^{5'"} is a C₁₋₃ alkyl group or a phenyl group, (f) a C₁₋₆ alkyl group which is substituted by a group of the formula: -NH-SO₂-R^{5""}, which R^{5""} is a C₁₋₆ alkyl group, and (g) a C₁₋₆ alkyl group which is substituted by a group of the formula: -NH-SO₂ -R^{5""'}, wherein R^{5""'} is a C₁₋₃ alkyl group.

As the group R⁴, an optionally substituted C₆₋₁₄ aryl group, an optionally substituted C₇₋₂₀ aralkyl group, an optionally substituted C₃₋₇ cycloalkyl group, an optionally substituted carboxyl group of the formula -CO-R¹¹ as mentioned above or an optionally substituted 5- to 13-membered heterocyclic group which contains 1 to 4 heteroatoms of oxygen, sulfur or nitrogen (5- or 6-membered heterocyclic group is preferable), are preferable.

The substituent of the above groups are the same as those of hydrocarbon residue as mentioned above.

As preferred examples of the substituents, mention is made of (1) halogen, (2) nitro, (3) cyano, (4) C₁₋₆ alkoxy which may optionally be substituted by C₁₋₆ alkoxy, carboxyl, halogen, C₁₋₆ alkyl-carbamoyl, 5 to 7 membered nitrogen-containing heterocyclic group, (5) C₇₋₁₃ aralkyloxy, (6) C₁₋₄ alkyl which may be substituted by hydroxy, oxo or C₁₋₃ alkoxy, (7) C₁₋₆ alkanoyl, (8) C₁₋₄ alkylthio, (9) C₂₋₆ alkenyloxy, (10) C₁₋₆ alkoxycarbonyl or (11) C₁₋₆ alkyl-aminocarbonyl.

As the group R⁴, preferred examples are a C₆₋₁₄ aryl group, a C₃₋₁₀ cycloalkyl group, a 5 to 13 membered heterocyclic group, or carboxyl group, each of these groups being optionally substituted, and an optionally substituted C₆₋₁₄ aryl group is more preferable.

In the group R⁴, as preferred examples of the substituents, mention is also made of C₁₋₆ alkoxy which may optionally substituted by a C₁₋₆ alkoxy, carboxyl, halogen, C₁₋₆ alkyl-carbamoyl or 5 to 7 membered nitrogen-containing heterocyclic group. Additional preferred examples of R⁴ are C₆₋₁₄ aryl which may be substituted by (1) C₁₋₆ alkoxy, which may be substituted by halogen or C₁₋₆ alkoxy or (2) C₁₋₆ alkylthio. A most preferred example of the group R⁴ is C₆₋₁₄ aryl which may optionally be substituted by an optionally substituted C₁₋₆ alkoxy, especially C₁₋₆ alkoxy which may optionally be substituted by C₁₋₆ alkoxy.

Still other preferred examples of the group R⁴ are phenyl which may be substituted by (1) C₁₋₄ alkoxy which may be substituted by C₁₋₆ alkoxy, carboxy, C₁₋₆ alkyl-carbamoyl, piperazinecarbonyl or halogen, (2) C₇₋₈ aralkyloxy, (3) C₁₋₄ alkyl which may optionally be substituted by hydroxy, oxo or C₁₋₃ alkoxy, especially C₁₋₄ alkyl which may optionally be substituted by C₁₋₃ alkoxy, (4) C₁₋₆ alkanoyl, (5) C₂₋₄ alkenyloxy, (6) C₁₋₆ alkoxy-carbonyl or (7) C₁₋₆ alkyl-carbamoyl.

In the above definitions, C₂₋₆ alkenyl is exemplified by vinyl, allyl, isopropenyl, butenyl, hexatrienyl, C₂₋₄ alkenyl is exemplified by vinyl, allyl, isopropenyl, butenyl.

C₆₋₁₄ aryl is exemplified by phenyl, naphthyl, anthryl, phenanthryl, acenaphthyl, anthracenyl, especially phenyl is most preferable.

C₇₋₈ aralkyl is exemplified by benzyl and phenethyl.

C₁₋₆ alkoxy is exemplified by methoxy, ethoxy, propoxy, isopropoxy, n-butoxy, isobutoxy, s-butoxy, t-butoxy, pentyloxy, hexyloxy, C₁₋₄ alkoxy is exemplified by methoxy, ethoxy, propoxy, isopropoxy, n-butoxy, isobutoxy, s-butoxy, t-butoxy. C₁₋₃ alkoxy is exemplified by methoxy, ethoxy, propoxy, isopropoxy.

Halogen is exemplified by fluorine, chlorine, bromine, iodine.

C₁₋₆ alkyl is exemplified by methyl, ethyl, n-propyl, isopropyl, n-butyl, isobutyl, t-butyl, pentyl, hexyl. C₁₋₄ alkyl is exemplified by methyl, ethyl, n-propyl, isopropyl, n-butyl, isobutyl, t-butyl. C₁₋₃ alkyl is exemplified by methyl, ethyl, n-propyl, isopropyl.

C₃₋₁₀ cycloalkyl is exemplified by cyclopropyl, cyclobutyl, cyclopentyl, cyclohexyl, cycloheptyl, cyclooctyl, cyclononyl. C₃₋₇ cycloalkyl is exemplified by cyclopropyl, cyclobutyl, cyclopentyl, cyclohexyl, cycloheptyl. C₃₋₆ cycloalkyl is exemplified by cyclopropyl, cyclobutyl, cyclopentyl, cyclohexyl.

C₁₋₆ acyl is exemplified by formyl and C₁₋₆ alkanoyl of the formula: -CO-R¹³, wherein R¹³ is methyl, ethyl, propyl, isopropyl, n-butyl, isobutyl, s-butyl, t-butyl, pentyl.

C₂₋₆ alkanoyl is exemplified by the formula: -CO-R¹³, wherein R¹³ has the same meaning as defined above. C₁₋₄ acyl is exemplified by formyl and the formula: -CO-R^{13'} (wherein R^{13'} is methyl, ethyl, n-propyl, isopropyl.).

Preferable five to seven-membered heterocyclic groups which contain 1 to 4 heteroatoms of oxygen, sulfur or nitrogen are exemplified by thienyl, furyl, pyrrolyl, pyrazolyl, thiazolyl, isothiazolyl, oxazolyl, isoxazolyl, imidazolyl, triazolyl, tetrazolyl, furazanyl, tetrahydrofuryl, pyridyl, pyrimidinyl, pyridazynyl, oxadiazolyl, tetrahydropyranyl, morpholinyl, thiomorpholinyl, pyrrolidinyl, pyrrolinyl, pyrazolidinyl, pyrazolinyl, imidazolidinyl, imidazolinyl, imidazolyl, 1,2,3-triazinyl, 1,2,3-triazolidinyl, 1,2,3-triazolyl, 1,2,3,4-tetrazolyl, piperidinyl, piperazinyl, hexamethyleneaminyl, oxazolidinyl or thiazolidinyl. As more preferable heterocyclic groups, mention is made of 5 to 6 membered heterocyclic groups. In particular, pyrrolidinyl, pyrazolinyl, pyrazolyl, piperidinyl, piperazinyl, morpholinyl and thiomorpholinyl are preferable.

In the above definition, the number of the substituent(s) is preferably 1 to 3.

The present compound (I) and their salts can be produced by per se known methods. Typically, the present compound can be produced by the processes described below.

(a) The compound (I') in which R¹ is hydrogen and R² is an optionally substituted hydrocarbon residue, that is to say compound (IV') or a salt thereof can be produced by cyclizing a compound of the following general formula (II') or a salt thereof with a base. 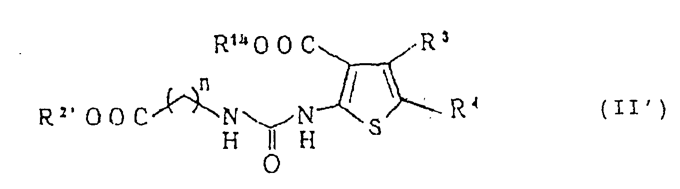 wherein R^{2'} represents an optionally substituted hydrocarbon residue; R³, R⁴, and n are as defined hereinbefore; R¹⁴ represents hydrogen or an optionally substituted hydrocarbon residue being the same as above.

This reaction is carried out in a solvent that does not interfere with the reaction. The solvent that can be used includes but is not limited to alcohols such as methanol, ethanol, isopropyl alcohol, etc. and ethers such as dioxane, tetrahydrofuran, etc.

The base mentioned above may for example be an alkali metal alkoxide, e.g. sodium methoxide, sodium ethoxide, sodium isopropoxide, etc., or an alkali metal hydride, e.g. sodium hydride.

The amount of the base with respect to compound (II') is about 1.1-5 molar equivalents, preferably about 1.5-3 equivalents.

The reaction temperature may range from about 10°C to the boiling point of the solvent used and is preferably about 25°C to the boiling point of the solvent.

The reaction time is several minutes to a few days and preferably about 10 minutes to 2 days.

(b) Compound (IV') or a salt thereof can be produced by cyclizing a compound of the following general formula (III) or a salt thereof in the presence of a base and subjecting the cyclization product to electrophilic substitution reaction for introducing a group of the formula -R⁴, where R⁴ is as defined hereinbefore. 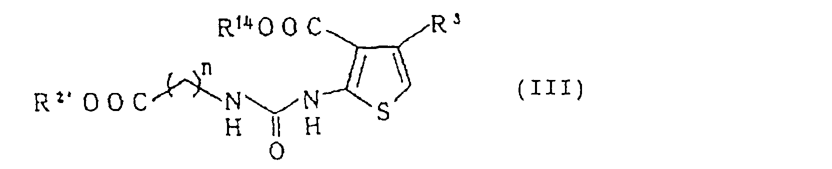 wherein R^{2'} represents an optionally substituted hydrocarbon residue; R³ is as defined hereinbefore; R¹⁴ represents hydrogen or an optionally substituted hydrocarbon residue; n represents a whole number of 1-3.

This cyclization reaction is conducted in a solvent that does not interfere with the reaction. The solvent that can be used includes but is not limited to alcohols such as methanol, ethanol, isopropyl alcohol, etc. and ethers such as dioxane, tetrahydrofuran, etc.

The base that can be used includes alkali metal alkoxides such as sodium methoxide, sodium ethoxide, sodium isopropoxide, etc. and alkali metal hydrides such as sodium hydride etc.

The proportion of the base with respect to compound (III) is about 1.1-5 molar equivalents and preferably about 1.5-3 equivalents.

The reaction temperature may range from about 10°C to the boiling point of the solvent used and is preferably about 25°C to the boiling point of the solvent.

The reaction time is several minutes to a few days and preferably about 10 minutes to 2 days.

This electrophilic substitution can be achieved by a per se known electrophilic substitution reaction. Specific examples of such reaction are the nitration reaction, e.g. the reaction using fuming nitric acid-concentrated sulfuric acid or sodium nitrate-concentrated sulfuric acid, acylation reaction, e.g. the reaction using an acid chloride-aluminum chloride, formylation reaction, e.g. the reaction using phosphorus oxychloride-N,N-dimethylformamide or N-methylformanilide, and halogenation reaction, e.g. the reaction using N-bromosuccinimide, bromine-pyridine, or sulfuryl chloride.

The electrophilic substitution reaction can be carried out under per se known reaction conditions. Typical sets of conditions are as follows. The nitration reaction is conducted in fuming nitric acid-concentrated sulfuric acid, sodium nitrate-concentrated sulfuric acid, or potassium nitrate-concentrated sulfuric acid at about 0-80°C. The acylation reaction is carried out using an alkanoyl chloride, e.g. acetyl chloride, propionyl chloride, etc, in a solvent that does not interfere with the reaction, e.g. nitrobenzene, nitromethane, carbon disulfide, etc, in the presence of a Lewis acid catalyst, e.g. aluminum chloride, titanium tetrachloride, etc, at about 0-100°C. The formylation reaction is carried out using phosphorus oxychloride-N,N-dimethylformamide/N-methylformanilide, oxalyl chloride-N,N-dimethylformamide/N-methylformanilide, thionyl chloride-N,N-dimethylformamide/N-methylformanilide in a solvent that does not interfere with the reaction, e.g. benzene, toluene, xylene, tetrahydrofuran, dioxane, 1,2-dichloroethane, etc, or in the absence of a solvent at about 15-130°C. The halogenation reaction is carried out using sulfuryl chloride, N-chlorosuccinimide, N-bromosuccinimide, bromine, chlorine, or iodine in a solvent that does not interfere with the reaction, e.g. dichloromethane, chloroform, carbon tetrachloride, 1,2-dichloroethane, pyridine, benzene, toluene, xylene, etc, at about 15-130°C.

The substituent group introduced by the above electrophilic substitution reaction can be subjected, where desired, to a functional group transformation reaction. This functional group transformation reaction can be carried out by the per se known transformation reaction. Specific examples of the reaction are reduction reaction, acylation reaction, sulfonylation reaction, alkylation reaction, diazo coupling reaction, Wittig reaction, halogenation reaction, halide-Grignard reaction, and coupling reaction with an organozinc reagent, an organoboron reagent or an organotin reagent.

(c) The compound (I') wherein R¹ represents an optionally substituted hydrocarbon residue and R² represents an optionally substituted hydrocarbon residue, that is to say compound (VI), or a salt thereof can be produced by reacting the compound (IV') or a salt thereof as prepared by the above procedure (a) or (b) with a compound of the general formula (V'): R¹-X (V'), wherein R¹ represents an optionally substituted hydrocarbon residue; X represents halogen, or a salt thereof.

The optionally substituted hydrocarbon residue mentioned for R¹ has the same meaning as defined hereinbefore. The halogen mentioned for X includes fluorine, chlorine, bromine, and iodine.

This reaction is conducted in a solvent that does not interfere with the reaction. The solvent that can be used includes ethers such as tetrahydrofuran, dioxane, etc., aromatic hydrocarbons such as benzene, toluene, xylene, etc., amides such as N,N-dimethylformamide, N,N-dimethylacetamide, etc., dimethyl sulfoxide, and so on. This reaction is preferably carried out under basic conditions, e.g. in the presence of potassium carbonate, sodium hydride, potassium hydride, potassium t-butoxide, or the like.

The proportion of compound (V') with respect to compound (IV') is about 1-5 molar equivalents and preferably about 1.1-2.5 equivalents.

When a base is used, its proportion is about 1-5 equivalents, preferably 1.1-3 equivalents, based on compound (IV').

The reaction temperature may range from about 10°C to the boiling point of the solvent used and is preferably about 20°C-130°C.

The reaction time ranges from several minutes to a few days and preferably from about 15 minutes to about 2 days.

(d) The hydroxyl group in the starting compound can be substituted by various kinds of groups. The reaction is carried out in an appropriate solvent, e.g. dimethylformamide (DMF), acetonitrile, acetone. To the solution of the starting compound is added halide such as alkyl halide, e.g. propyl iodide, isobutyl iodide, ethybromo acetate, or aralkyl halide, e.g. benzylchlolide. The mixture is stirred at 0 to 40°C for 2 to 18 hours.

For example, in the case of ethyl bromoacetate, the obtained acetic acid ester is hydrolyzed in an adequate solvent and base, e.g. iN NaOH solution in ethyl alcohol, at room temperature for 2 to 12 hours. The acetic acid compound is dissolved in an adequate solvent; e.g. tetrahydrofuran (THF). To the solution is added isobutyl chloroformate in the presence of an adequate base, e.g. Et₃N, and the reaction is carried out at 0°C for 1 to 4 hours. To the solution is added adequate amine derivatives, e.g. methylamine, propylamine, piperidine. The reaction is carried out at 0°C to room temperature for 1 to 12 hours.

Said starting compound which has a hydroxyl group is produced by acid-hydrolysis of a compound such as one having an alkoxy group. The acid hydrolysis is carried out in a conventional manner such as by adding 1N hydrochloric acid in an appropriate solvent such as tetrahydrofuran or alcohol, e.g. methanol, ethanol, at 0°C to room temperature for one to 10 hours.

(e) The present compound (I'), wherein WR⁴ is an alkanoyl- phenyl group can be produced by the introduction of a alkanoyl-phenyl group to the halogenated compound (WR⁴=Br). The halogenated compound is obtained by the halogenation reaction with the starting compound (WR⁴=H). The halogenation is carried out in an adequate solvent, e.g. carbontetrachloride or chloroform. To the solution is added N-bromosuccinimide and catalytic amount of 2,2'-azobis- (isobutyronitrile). The reaction is carried out at 100 to 120°C for 1 to 4 hours. The introduction reaction of alkanoyl phenyl group is carried out in an appropriate degased solvent, e.g. dimethoxyethane (DME). To the solution is added alkanoyl phenyl borate, palladium compound, e.g. Pd(PPh₃)₄(Ph=phenyl) and sodium carbonate (2M, Na₂CO₃). The alkanoyl phenyl borate is synthesized by the reaction of alkanoyl phenyl bromide with adequate borate, e.g. (i-PrO)₃B(Pro=propyl) in the presence of adequate base, e.g. BuLi (Bu=butyl). The introduction reaction is carried out at room temperature to 120°C for 1 to 12 hours under inert gas atmosphere.

(f) The present compound (I'), wherein WR⁴ is alkylphenyl group can be produced by the similar manner as shown in (e) with alkyl phenyl borates instead of alkanoyl phenyl borates.

Any other group in the compound can be introduced by any known per se known methods.

(g) The present compound (I), wherein R³ is an alkoxycarbonyl group, can be produced by introducing a cyano group, and then subjecting the obtained compound to esterification.

In the reaction of the introduction of cyano group, the starting compound is dissolved in an appropriate solvent, e.g. dimethylsulfoxide (DMSO), and to the solution is added sodium cyanide. The reaction is carried out at 40 to 60°C for 2 to 12 hours.

The esterification reaction is carried out in an appropriate solvent such as ethyl alcohol. The reaction is conducted by mixing the starting compound and alcohol solution, e.g. ethyl alcohol, saturated with hydrochloric acid. The reaction is carried out at 80 to 120°C for 12 to 48 hours.

(h) The present compound (I'), wherein R³ is an alkyl group which is substituted by a group -NH-SO₂-R⁵, wherein R⁵ is the same meaning as defined above, can be synthesized by (i) halogenation of this alkyl group and (ii) nucleophilic substitution of this halogen with a sulfonamide compound in the presence of appropriate base, e.g. sodium hydride.

The halogenation is carried out in an appropriate solvent, e.g. carbon tetrachloride. To the solution is added N-bromosuccinimide or catalytic amount of 2,2'-azobis(isobutyronitrile). The reaction is carried out at 100 to 120°C for 1 to 4 hours.

The nucleophilic substitution reaction is carried out in a similar manner as described in the above process (P) on the reaction of the compound (IV') and (V'). Particularly, in an appropriate solvent such as N,N-dimethylformamide (DMF). To the solution is added sodium hydride washed with n-hexane and sulfonamide derivatives, e.g. methanesulfonamide, ethanesulfonamide, benzenesulfonamide. The reaction is carried out at 0 to 40°C for 1 to 24 hours.

(i) The compound (I') wherein R² is hydrogen, that is to say compound (VII) or (VII'), or a salt thereof, can be obtained by subjecting the compound (IV') or (VI), or a salt thereof, as produced in the above manner to a reaction for conversion of R² to hydrogen.

The reaction for converting R² to hydrogen or from esters to carboxylic groups may for example be a alkali-hydrolysis reaction. This hydrolysis reaction is conducted by reacting compound (IV') or (VI), or a salt thereof, with a base in a solvent that does not interfere with the reaction. The solvent that can be used for this reaction includes alcohols such as methanol, ethanol, isopropyl alcohol, etc., ethers such as tetrahydrofuran, dioxane, etc., amides such as N,N-dimethylformamide, N,N-dimethylacetamide, etc., and dimethyl sulfoxide, among others. The base that can be used includes alkali metal hydroxides such as lithium hydroxide, sodium hydroxide, potassium hydroxide, etc., alkaline earth metal hydroxides such as calcium hydroxide, barium hydroxide, etc., and alkali metal carbonates such as potassium carbonate, sodium carbonate, etc. The proportion of the base to compound (IV') or (VI) is about 1-10 molar equivalents and preferably about 1.5-5 equivalents. The reaction temperature may range from about 10°C to the boiling point of the solvent used and is preferably about 15°-100°C. The reaction time is several minutes to a few days and preferably about 15 minutes to two days.

The compound of the item (28) aforementioned can be produced by subjecting a starting compound (I') in which R³ is alkoxycarbonyl-methyl to an alkali-hydrolysis as mentioned above.

(j) The present compound (I'), wherein R² is the optionally substituted hydrocarbon residue such as pivaloyloxymethyl or 1-(cyclohexyloxycarbonyloxy)ethyl can be synthesized by the condensation reaction of the compound (I',R²=H) with chloride agents (e.g. pivaloyloxymethyl chloride, 1-(cyclohexyloxycarbonyloxy)ethyl-1-chloride) or acid anhydride agents, e.g. pivalic anhydride, in an appropriate solvent, e.g. dimethylformamide (DMF), in the presence of adequate base (e.g. K₂CO₃) and potassium iodide (KI). The reaction is carried out at 0°C to room temperature for 2 to 24 hours.

The starting compounds (II') and (III), as well as salts thereof, which are to be employed in the above production processes can be produced typically by the following alternative processes A and B.

### 1. Process A

In this process, either a compound of the general formula (VIII) or a salt thereof or a compound of the general formula (VIII') or a salt thereof is reacted with an isocyanic acid ester derivative. 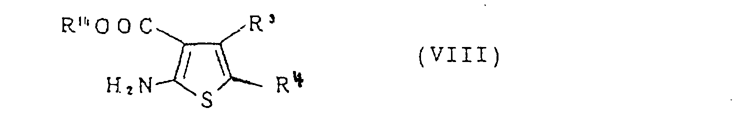 wherein R³, R⁴, R¹⁴, and n are as defined hereinbefore, 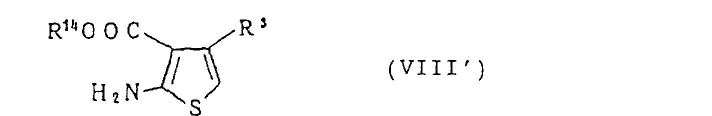 wherein R³ and R¹⁴ are as defined as hereinbefore.

The isocyanic acid derivative mentioned above may for example be an isocyanate derivative of the formula R¹⁴OOC-(CH₂)ₙ-NCO, wherein R¹⁴ and n are as defined hereinbefore.

The reaction of compound (VIII) or compound (VIII'), or a salt thereof, with said isocyanate derivative is carried out in a solvent which does not interfere with the reaction, e.g. tetrahydrofuran, pyridine, dioxane, benzene, dichloromethane, 1,2-dichloroethane, toluene, xylene, etc, at about 15-130°C and preferably at about 25-130°C.

The isocyanate derivative is used in a proportion of about 1-5, preferably about 1.1-2.5 molar equivalents, relative to compound (VIII) or (VIII').

The reaction time is several minutes to a few days and preferably about 15 minutes to about 2 days.

### 2. Process B

This process comprises reacting compound (VIII) or (VIII'), or a salt thereof, with phosgene or the equivalent, e.g. triphosgene of bis(trichloromethyl) carbonate or the like, diphosgene of trichloromethyl chloroformate or the like, etc, to give the isocyanate derivative and adding an amine, e.g. a compound of the formula R¹⁴OOC-(CH₂)n-NH₂, where R¹⁴ and n are as defined hereinbefore.

The reaction between compound (VIII) or (VIII'), or a salt thereof, and phosgene or the equivalent is conducted in a solvent that does not interfere with the reaction, e.g. dioxane, tetrahydrofuran, benzene, toluene, xylene, 1,2-dichloroethane, chloroform, etc, at about 15-130°C and preferably at about 25-130°C.

Phosgene or the equivalent thereof is used in a proportion of about 0.5-2 molar equivalents, preferably about 0.9-1.1 equivalents, with respect to compound (VIII) or (VIII').

The reaction time is several minutes to a few days and preferably about 15 minutes to about two days.

The amine addition reaction is carried out in a solvent that does not interfere with the reaction, e.g. pyridine, tetrahydrofuran, dioxane, benzene, dichloromethane, 1,2-dichloroethane, toluene, xylene, etc, at about 15-130°C and preferably at about 25-130°C.

The amine is used in a proportion of about 1-5 molar equivalents, preferably about 1.1-3 equivalents, with respect to compound (VIII) or (VIII').

The reaction time is several minutes to a few days and preferably about 15 minutes to about two days.

The compound (VIII) or a salt thereof for use in the above reaction can be produced by the following process.

A ketone having an active methylene group, e.g. a compound (IX) of the formula R³-CO-CH₂-WR⁴, where R³, R⁴ and W are as defined hereinbefore, is reacted with a cyanoacetic ester derivative and sulfur according to the method of K. Gewald, E. Schinke and H. Bettcher, Chem. Ber., 99, 94-100, 1966, to give compound (VIII) or a salt thereof. Thus, the above-mentioned ketone and the cyanoacetate derivative are heated together under reflux in a solvent that does not interfere with the reaction, e.g. benzene, toluene, etc, in the presence of acetic acid and ammonium acetate to give the alkylidenecyanoacetate derivative which is then heated in a solvent that does not interfere with the reaction, e.g. methanol, ethanol, etc, in the presence of sulfur and a base, e.g. an organic base such as triethylamine, ethyldiisopropylamine, dimethylaminopyridine, etc, at a temperature of about 50-80°C to give 2-aminothiophene derivative i.e. Compound (VIII).

Compound (VIII') can be synthesized by the method of K. Gewald (Chem. Ber., 98, 3571-3577 (1965) (K. Gewald) and Chem. Ber., 99, 2712-2715 (1966) (K. Gewald and E. Schinke).

In this specification, "the present compound" means the compounds of this invention, such as the compound (I), the compound (I') and the compound of the above item (28).

The salt of the present compound thus obtained is preferably a physiologically acceptable acid addition salt. Such addition salt may for example be any of salts with inorganic acids, e.g. hydrochloric acid, hydrobromic acid, nitric acid, sulfuric acid, phosphoric acid, etc, and salts with organic acids, e.g. formic acid, acetic acid, trifluoroacetic acid, fumaric acid, oxalic acid, tartaric acid, maleic acid, citric acid, succinic acid, malic acid, methanesulfonic acid, benzenesulfonic acid, etc. Where the present compound of the invention has an acidic group such as -COOH, the present compound may form salts with inorganic bases, e.g. alkali metals or alkaline earth metals such as sodium, potassium, calcium, magnesium, etc, or ammonia, or organic bases, e.g. trimethylamine, triethylamine, pyridine, picoline, ethanolamine, diethanolamine, triethanolamine, dicyclohexylamine, N,N'-dibenzylethylenediamine, etc.

The compound or salt of the invention as produced by the above-described technology can be isolated and purified by the conventional procedures such as recrystallization, distillation, and chromatography, among other fractionation techniques. Where the present compound is obtained as a free compound, it can be converted to a salt by a per se known method or any method analogous therewith. Conversely where a salt is obtained, it can be converted to the free compound or a different salt by a per se known method or any method analogous therewith.

The salts of the compounds (II) to (IX) can also be salts similar to the salts of compound (I).

Where the present compound or salt of the invention is an optically active compound, it can be fractionated into the d- and ℓ-compounds by a conventional optical resolution technique.

The present compound has only a low toxic potential and can, therefore, be safely used.

The endothelin antagonist composition of the present invention has remarkably potent endothelin receptor antagonist activity and can be administered as an endothelin antagonist to mammals, e.g. rat, mouse, rabbit, cat, dog, bovine, equine, and human being. Specifically, it can be used safely as a therapeutic drug for acute renal failure, myocardial infarction, liver disorder, angina pectoris, cerebral infarction, cerebrovasospasm, hypertension, kidney disease, asthma, ectopic angina, Raynaud's syndrome, pulmonary hypertension, surgical shock, chronic cardiac insufficiency, atherosclerosis, cardiac hypertrophy and migraine, among other diseases, as a prophylactic or therapeutic drug for organ, e.g. liver, surgery- or transplant-associated organic hypofunction, or as a prophylactic agent for post-PTCA vascular restenosis. Particularly, the composition is of great use as a therapeutic drug for acute renal failure, myocardial infarction, hepatic disorder, hypertension, and pulmonary hypertension, as a prophylactic or therapeutic drug for organ, e.g. liver, surgery- or transplant-associated organic hypofunction, or as a prophylactic drug for post-PTCA vascular restenosis. Furthermore, the compound of the present invention can be used as an inhibitor for vasoconstriction, such as an inhibitor for vasoconstriction of coronary artery, coronary vein, cerebrovascular system or pulmonary vascular system.

When the present compound or a salt thereof is to be administered to a human being, the compound as such or in the form of a pharmaceutical composition formulated with a suitable pharmacologically acceptable carrier, excipient or diluent can be safely administered orally or non-orally.

The pharmaceutical composition mentioned above may be provided in various dosage forms such as oral dosage forms, e.g. powders, granules, capsules, tablets, etc., injections, drip injections, dosage forms for external application, e.g. nasal dosage forms and transdermal drug delivery systems, and suppositories, e.g. rectal suppositories, vaginal suppositories.

These dosage forms can be manufactured by the established pharmaceutical procedures.

The present compound or salt of the invention can be formulated with a dispersant, e.g. Tween 80, Atlas Powder Co., U.S.A., HOC 60, Nikko Chemicals Co., polyethylene glycol, carboxymethylcellulose, sodium alginate, etc., a preservative, e.g methyl p-hydroxybenzoate, propyl p-hydroxybenzoate, benzyl alcohol, etc., an isotonizing agent, e.g. sodium chloride, mannitol, sorbitol, glucose, etc., and other additives to provide an aqueous injection, or dissolved, suspended or emulsified in vegetable oil, e.g. olive oil, sesame oil, cottonseed oil, corn oil, etc., propylene glycol, or the like to provide an oily injection.

For the manufacture of oral dosage forms, the present compound or salt of the invention is formulated with, for example, an excipient, e.g. lactose, sucrose, starch, etc., a disintegrator, e.g. starch, calcium carbonate, etc., a binder, e.g. starch, gum arabic, carboxymethylcellulose, polyvinylpyrrolidone, hydroxypropylcellulose, etc., and/or a lubricant, e.g. talc, magnesium stearate, polyethylene glycol 6000, etc., and compressed in the per se conventional manner. Where necessary, for masking the taste or insuring enteric or sustained release, the compressed composition can be coated by the per se known technique to provide an oral dosage form. The coating agent that can be used includes but is not limited to hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit, Rohm & Haas Co., Germany; methacrylic-acrylic acid copolymer, and pigments, e.g. red iron oxide, titanium dioxide, etc.. In the manufacture of an enteric release dosage form, it is preferable to provide an intermediate phase between an enteric phase and a drug-containing phase for phase-to-phase isolation.

For the manufacture of dosage forms for external application, the present compound or salt of the invention can be processed into solid, semisolid or liquid preparations. To provide a solid preparation, for instance, the present compound or a salt thereof is used as it is or in the form of a powdery composition formulated with an excipient, e.g. glycol, mannitol, starch, microcrystalline cellulose, etc., a thickener, e.g. natural gums, cellulose derivatives, acrylic polymers, etc., and other additives. A liquid preparation can be substantially similar to the injection mentioned above and may be an oily or aqueous suspension. The semisolid preparation can be an aqueous or oleaginous gel or ointment. To any of these preparations, a pH control agent, e.g. carbonic acid, phosphoric acid, citric acid, hydrochloric acid, sodium hydroxide, etc. and an antiseptic, e.g. p-hydroxybenzoic esters, chlorobutanol, benzalkonium chloride, etc. can be added.

For the production of suppositories, the present compound or salt of the invention can be processed into oleaginous or hydrous solid, semisolid or liquid suppositories in accordance with per se known production procedures. The oleaginous base that can be used for the above composition includes higher fatty acid glycerides, e.g. caccao butter, witepsols, Dynamite Nobel, Germany, medium fatty acid glycerides, e.g. miglyols, Dynamite Nobel, Germany, etc., and vegetable oils, e.g. sesame oil, soybean oil, cottonseed oil, etc.. The water-soluble base includes polyethylene glycols, propylene glycol, and the hydrogel base that can be used includes natural gums, cellulose derivatives, vinyl polymers, acrylic polymers, and so on.

The daily dosage of the present compound varies with the severity of illness, the recipient's age, sex, body weight, and sensitivity, administration time and interval, the property, recipe, and type of dosage form, and species of active ingredient, among other variables, and cannot be stated in general terms. Usually, however, the recommended dosage is about 0.01-10 mg, preferably about 0.03-3 mg, per kilogram body weight of the mammal and the above amount is usually administered once or in up to 4 divided doses a day.

The compound of the present invention has particularly high endothelin receptor antagonist activity. Moreover, the compound is highly amenable to oral administration and features a sustained action.

The following examples are intended to describe the present invention in further detail and should by no means be construed as defining the scope of the invention.

The ¹H-NMR spectra shown were determined with a Varian Gemini 200 (200 MHz) spectrometer or Bruker AM-500 (500 MHz) spectrometer using tetramethylsilane as internal standard and all δ values were expressed in ppm.

The symbols used have the following meanings.
s: singlet, d: doublet, t: triplet, q: quartet, dt: double triplet, m: multiplet, br: broad, J: coupling constant.

### Reference Example 1

Production of ethyl 2-amino-4-methyl-5-(4-methoxyphenyl)thiophene-3-carboxylate:

A mixture of 4-methoxyphenylacetone (16.5 g, 0.10 mol), ethyl cyanoacetate (12.2 g, 0.10 mol), ammonium acetate (1.55 g, 20 mmol), acetic acid (4.6 ml, 80 mmol), and benzene (20 ml) was refluxed for 24 hours, with the byproduct water being removed with a Dean-Stark trap. After cooling, the reaction mixture was concentrated under reduced pressure and the residue was distributed between dichloromethane and sodium hydrogen carbonate-water. The organic layer was washed with NaCl-water and dried (MgSO₄) and the solvent was distilled off under reduced pressure. The residue was dissolved in ethanol (30 ml), and sulfur (3.21 g, 0.10 mol) and diethylamine (10.4 ml, 0.10 mol) were added. This mixture was stirred at 50-60°C for 2 hours and then concentrated and the residue was extracted with ethyl acetate. The extract was washed with NaCl-water and dried (MgSO₄) and the solvent was distilled off under reduced pressure. The residue was purified by silica gel column chromatography and crystallized from ether-hexane to provide light-yellow platelets (11.5 g, 40%). m.p. 79-80°C.

| Elemental analysis for C₁₅H₁₇NO₃S | | | | |
|---|---|---|---|---|
| | C (%) | H (%) | N (%) | S (%) |
| Calcd. | 61.83; | 5.88; | 4.81; | 11.01 |
| Found | 61.81; | 5.75; | 4.74; | 10.82 |

¹H-NMR (200 MHz, CDCl₃) δ: 1.37 (3H, t, J=7.1 Hz), 2.28 (3H, s), 3.83 (3H, s), 4.31 (2H, q, J=7.1 Hz), 6.05 (2H, br s), 6.91 (2H, d, J=8.8 Hz), 7.27 (2H, d, J=8.8 Hz).
IR (KBr): 3426, 3328, 1651, 1586, 1550, 1505, 1485 cm⁻¹.
FAB-MS m/z: 291 (M⁺).

### Reference Example 2

(1) Production of ethyl 2,4(1H,3H)-dioxo-6-(4-methoxyphenyl)thieno[2,3-d]pyrimidine-3-acetate:

To a pyridine solution (30 ml) of the ethyl 2-amino-4-methyl-5-(4-methoxyphenyl)thiophene-3-carboxylate obtained in Reference Example 1 (8.00 g, 27.0 mmol) was added ethyl isocyanatoacetate (4.54 ml, 40.5 mmol) dropwise and the mixture was stirred at 50°C for 2 hours. This reaction mixture was concentrated to dryness and the residue was distributed between ethyl acetate and ammonium chloride-water. The aqueous layer was extracted with ethyl acetate. The extracts were combined, washed with NaCl-water, and dried (MgSO₄) and the solvent was distilled off under reduced pressure. The residue was suspended in ethanol (100 ml) and following addition of potassium tert-butoxide (6.06 g, 54.0 mmol), the suspension was stirred at room temperature for 3 hours. To this reaction mixture was added 1N-HCl (50 ml) with ice-cooling and the ethanol was distilled off under reduced pressure. The resulting crystals were collected by filtration, rinsed with water-ethanol, and dried in vacuo over phosphorus pentoxide to provide white powders (11.0 g, 96%). For use as a sample for elemental analysis, the above powders were recrystallized from ethanol to provide colorless crystals. m.p. 164-165°C.

(2) Using ethyl 2-amino-4-methylthiophene-3-carboxylate, the procedure of Reference Example 2 (1) was repeated to provide ethyl 2,4(1H,3H)-dioxo-5-methylthieno[2,3-d]pyrimidine-3-acetate. Yield 94%, amorphous.

(3) To a solution of ethyl 2,4(1H,3H)-dioxo-5-methylthieno[2.3-d]pyrimidine-3-acetate obtained in the above item (2) in chloroform was added N-bromosuccinimide. Then the mixture was refluxed for 2 hours to provide ethyl 2,4(1H,3H)-dioxo-5-methyl-6-bromothieno[2,3-d]pyrimidine-3-acetate. Yield 86%, amorphous.

### Reference Example 3

Production of ethyl 2,4(1H,3H)-dioxo-6-(4-hydroxyphenyl)-5-methylthieno[2,3-d]pyrimidine-3-acetate:

To an ice-cooled mixture of aluminum chloride (2.90 g, 21.7 mmol), methyl disulfide (2.45 ml, 27.2 mmol), and dichloromethane (60 ml) was added a solution of the compound obtained in Reference Example 2 (2.0 g, 5.34 mmol) in dichloromethane (40 ml) dropwise and the mixture was stirred at room temperature for 20 hours. The reaction mixture was then poured in ice-water and the dichloromethane was distilled off under reduced pressure. This suspension was extracted with ethyl acetate and the extract was washed with NaCl-water and dried (MgSO₄). The solvent was then distilled off under reduced pressure and the residue was purified by silica gel column chromatography to provide white powders (1.64 g, 85%). For use as a sample for elemental analysis, the powders were recrystallized from ethyl acetate to provide colorless crystals. m.p. 240-242°C.

| Elemental analysis for C₁₇H₁₆N₂O₅S•0.1H₂O | | | |
|---|---|---|---|
| | C (%) | H (%) | N (%) |
| Calcd. | 56.38; | 4.51; | 7.73 |
| Found | 56.28; | 4.48; | 7.64 |

¹H-NMR (200 MHz, DMSO-d₆) δ: 1.22 (3H, t, J=7.1 Hz), 2.37 (3H, s), 4.15 (2H, q, J=7.1 Hz), 4.59 (2H, s), 6.85 (2H, d, J=8.6 Hz), 7.26 (2H, d, J=8.6 Hz), 9.73 (1H, s), 12.39 (1H, s).
IR (KBr): 3356, 2992, 1720, 1690, 1667, 1611, 1593, 1568, 1537, 1502 cm⁻¹.

### Reference Example 4

Production of ethyl 2,4(1H,3H)-dioxo-6-(4-hydroxyphenyl)-1-(2-methylthiobenzyl)-5-methylthieno[2,3-d]-pyrimidine-3-acetate:

To a solution of the compound obtained in Reference Example 3 (0.60 g, 1.66 mmol) in pyridine (8 ml) was added acetic anhydride (3 ml, 31.8 mmol) and the mixture was stirred at room temperature for 3 hours. This reaction mixture was concentrated and the residue was distributed between ethyl acetate and diluted hydrochloric acid. The aqueous layer was extracted with ethyl acetate. The organic layers were combined, washed with NaCl-water, and dried (MgSO₄) and the solvent was distilled off under reduced pressure. The residue was purified by silica gel column chromatography to provide a white amorphous solid (0.57 g). To a solution of this amorphous solid in dimethylformamide (5 ml) were added potassium carbonate (0.38 g, 2.75 mmol) and 2-methylthiobenzyl chloride (0.65 g, 4.15 mmol) and the mixture was stirred at room temperature for 22 hours. This reaction mixture was concentrated and the residue was distributed between ethyl acetate and NaCl-water. The aqueous layer was extracted with ethyl acetate. The organic layers were combined, washed with NaCl-water, and dried (MgSO₄) and the solvent was distilled off under reduced pressure. The residue was purified by silica gel column chromatography to provide a white amorphous solid (0.60 g). This amorphous solid was dissolved in methanol (18 ml)-tetrahydrofuran (12 ml) and a solution of potassium carbonate (0.313 g, 2.26 mmol) in water (8 ml) was added dropwise. The mixture was stirred at room temperature for 30 minutes and after 1N-hydrochloric acid (5 ml) was added under ice-cooling, the mixture was extracted with ethyl acetate. The extract was washed with NaCl-water and dried (MgSO₄), and the solvent was distilled off under reduced pressure. The residue was crystallized from ether to provide colorless crystals (4.33 g, 78%). m.p. 177-178°C.

| Elemental analysis for C₂₅H₂₄N₂O₅S₂•1/10H₂O | | | |
|---|---|---|---|
| | C (%) | H (%) | N (%) |
| Calcd. | 60.25; | 4.89; | 5.62 |
| Found | 60.09; | 4.66; | 5.57 |

¹H-NMR (200 MHz, CDCl₃) δ: 1.32 (3H, t, J=7.2 Hz), 2.45 (3H, s), 2.52 (3H, s), 4.28 (2H, q, J=7.2 Hz), 4.87 (2H, s), 5.28 (2H, s), 5.75 (1H, s), 6.78 (2H, d, J=8.6 Hz), 6.97-7.14 (4H, m), 7.21-7.34 (2H, m).
IR (KBr): 3346, 2978, 1752, 1700, 1651, 1613, 1591, 1564, 1535, 1481 cm⁻¹.

### Reference Example 5

(1) Using the compound obtained in Reference Example 3, the procedure of Reference Example 4 was repeated except that 2-chloro-6-fluorobenzyl chloride was used in lieu of 2-methylthiobenzyl chloride to provide ethyl 2,4(1H,3H)-dioxo-6-(4-hydroxyphenyl)-1-(2-chloro-6-fluorobenzyl)-5-methylthieno[2,3-d]pyrimidine-3-acetate. Yield 59%, amorphous.

(2) Using the compound obtained in Reference Example 2 (3), the procedure of Reference Example 4 was repeated to provide ethyl 2,4(1H,3H)-dioxo-1-(2-methylthiobenzyl)-5-methylthieno[2,3-d]pyrimidine-3-acetate. Yield 87%, amorphous.

### Reference Example 6

Production of ethyl 2,4(1H,3H)-dioxo-6-(4-methoxy-methoxyphenyl)-1-(2-methylthiobenzyl)-5-methylthieno[2,3-d]pyrimidine-3-acetate:

To a suspension of sodium hydride (60% in oil, 500 mg, 12.5 mmol) in dimethylformamide (20 ml) was added a solution of the compound obtained in Reference Example 4 (2.0 g, 3.7 mmol) in dimethylformamide (30 ml) dropwise in a nitrogen gas stream under ice-cooling. The mixture was stirred at the same temperature for 30 minutes and, then, chloromethyl methyl ether (1.0 g, 12.4 mmol) was added dropwise. This mixture was stirred at room temperature for 16 hours and then concentrated, and the residue was distributed between ethyl acetate and aqueous ammonium chloride solution. The aqueous layer was extracted with ethyl acetate. The extracts were combined, washed with NaCl-water, and dried (MgSO₄) and the solvent was distilled off under reduced pressure. The residue was purified by silica gel column chromatography and recrystallized from ethyl acetate-hexane to provide colorless crystals (1.05 g, 59%). m.p. 133-134°C.

| Elemental analysis for C₂₇H₂₈N₂O₆S | | | |
|---|---|---|---|
| | C (%) | H (%) | N (%) |
| Calcd. | 63.76; | 5.55; | 5.51 |
| Found | 63.48; | 5.62; | 5.37 |

¹H-NMR (300 MHz, CDCl₃) δ: 1.30 (3H, t, J=7.1 Hz), 1.43 (3H, t, J=7.0 Hz), 2.49 (3H, s), 3.87 (3H, s), 4.05 (2H, q, J=7.0 Hz), 4.25 (2H, q, J=7.1 Hz), 4.83 (2H, s), 5.24 (2H, s), 6.86-6.94 (4H, m), 7.09-7.14 (1H, m), 7.22-7.31 (3H, m).
IR (KBr): 2984, 1758, 1707, 1665, 1607, 1562, 1535, 1477 cm⁻¹.

### Reference Example 7

The compound obtained in Reference Example 5 was reacted with 2-chloro-6-fluorobenzyl chloride in lieu of chloromethyl methyl ether to provide ethyl 2,4(1H,3H)-dioxo-6-(4-isobutoxyphenyl)-1-(2-chloro-6-fluorobenzyl)-5-methylthieno[2,3-d]pyrimidine-3-acetate. Yield 29%, amorphous.

### Reference Example 8

Production of ethyl 2,4(1H,3H)-dioxo-5-bromomethyl-1-(2-methylthiobenzyl)-6-(4-methoxymethoxyphenyl)thieno[2,3-d]pyrimidine-3-acetate:

A mixture of the compound obtained in Reference Example 6 (1.20 g, 2.22 mmol), N-bromosuccinimide (0.4 g, 2.25 mmol), α,α'-azobisisobutyronitrile (50 mg), and carbon tetrachloride (50 ml) was refluxed for 4 hours. After cooling, the insolubles were filtered off and the filtrate was diluted with dichloromethane. The organic layer was washed with NaCl-water and dried (MgSO₄) and the solvent was distilled off under reduced pressure to provide a yellow amorphous solid (2.0 g).
¹H-NMR (300 MHz, CDCl₃) δ: 1.28 (3H, t, J=7.2 Hz), 2.53 (3H, s), 3.50 (3H, s), 4.26 (2H, q, J=7.2 Hz), 4.80 (2H, s), 4.89 (2H, s), 5.22 (2H, s), 5.36 (2H, s), 7.00-7.50 (8H, m).

### Reference Example 9

Production of ethyl 2,4(1H,3H)-dioxo-6-(4-methoxyphenyl)-1-(2-methylthiobenzyl)-5-methylthieno[2,3-d]-pyrimidine-3-acetate:

To a solution of the compound obtained in Reference Example 2 (2.0 g, 5.35 mmol) in dimethylformamide (25 ml) were added potassium carbonate (1.1 g, 7.98 mmol), potassium iodide (catalyst amount), and 2-methylthiobenzyl chloride (1.2 g, 6.96 mmol) and the mixture was stirred at room temperature for 18 hours. This reaction mixture was concentrated and the residue was distributed between ethyl acetate and NaCl-water. The aqueous layer was extracted with ethyl acetate. The organic layers were combined, washed with NaCl-water, and dried (MgSO₄) and the solvent was distilled off under reduced pressure. The residue was purified by silica gel column chromatography to provide a light-yellow amorphous solid (1.8 g, 66%). Recrystallization from ether gave colorless crystals. m.p. 144-145°C.

### Reference Example 10

Starting with the compound obtained in Reference Example 2, the procedure of Reference Example 9 was otherwise repeated to provide ethyl 2,4(1H,3H)-dioxo-6-(4-methoxyphenyl)-1-(2-chloro-6-fluorobenzyl)-5-methylthieno[2,3-d]pyrimidine-3-acetate. Yield 95%, amorphous.

### Reference Example 11

Starting with the compounds obtained in Reference Examples 9 and 10, respectively, the procedure of Reference Example 8 was repeated to provide the following compounds.
Compound 1: Ethyl 2,4(1H,3H)-dioxo-6-(4-methoxyphenyl)-1-(2-methylthiobenzyl)-5-bromomethylthieno[2,3-d]pyrimidine-3-acetate. Yield 95%, amorphous.
Compound 2: Ethyl 2,4(1H,3H)-dioxo-6-(4-methoxyphenyl)-1-(2-chloro-6-fluorobenzyl)-5-bromomethylthieno[2,3-d]pyrimidine-3-acetate. Yield 100%, amorphous.
Compound 3: Ethyl 2,4(1H,3H)-dioxo-6-(4-isobutoxyphenyl)-1-(2-chloro-6-fluorobenzyl)-5-bromomethylthieno[2,3-d]pyrimidine-3-acetate. Yield 60%, amorphous.

### Reference Example 12

In accordance with the similar manner of Reference Examples 6 and 8 the following compounds were obtained.

Compound 1: Ethyl 2,4(1H,3H)-dioxo-5-bromomethyl-1-(2-methylthiobenzyl)-6-(4-propoxyphenyl)thieno[2,3-d]pyrimidine-3-acetate. amorphous.

### Reference Example 13

Production of ethyl {2,4(1H,3H)-dioxo-5-methyl-1-(2-methylthiobenzyl)-6-(4-(2-methoxyethyl)phenyl)-thieno[2,3-d]pyrimidine-3-acetate:

To a mixture of ethyl {2,4(1H,3H)-dioxo-6-bromo-5-methyl-1-(2-methylthiobenzyl)thieno[2,3-d]pyrimidine-3-acetate (1.0 g, 2.07 mmol) obtained in Reference Example 5(2), 4-(methoxyethylphenyl)boronic acid (1.0 g, 5.56 mmol), and 2M sodium carbonate (5.2 ml, 10.4 mmol) in 1,2-dimethoxyethane (50 ml) was added Pd(PPh₃)₄(Ph denotes phenyl) (358 mg, 0.31 mmol) under argon atmosphere. The mixture was stirred under reflux for 5 hour and filtered through celite. The filterate was partitioned between ethyl acetate and brine. The aqueous phase was separated and extracted with ethyl acetate. The combined extracts were washed with brine, dried over MgSO₄ and concentrated in vacuo. The residue was chromatographed on silica gel with ethyl acetate and n-hexane (1:5 - 1:3) to give the product (860 mg, 77%) as colorless amorphous solid. Recrystallization from ethyl acetate and nhexane gave product (594 mg) as colorless powder, m.p. 126-128°C.

### Reference Example 14

Production of ethyl 2,4(1H,3H)-dioxo-5-bromomethyl-1-(2-methylthiobenzyl)-6-(4-(2-methoxyethyl)phenyl)thieno[2,3-d]pyrimidine-3-acetate:

A mixture of ethyl 2,4(1H,3H)-dioxo-5-methyl-1-(2-methylthiobenzyl)-6-(4-(2-methoxyethyl)phenyl)thieno [2,3-d]pyrimidine-3-acetate (600 mg, 1.11 mmol) obtained in Reference Example 13, N-bromosuccinimide (198 mg, 1.11 mmol) and 2,2'-azobisisobutyronitrile (18 mg, 0.11 mmol) in chloroform (30 ml) was stirred under reflux for 1.5 hour. The mixture was partitioned between CH₂Cl₂+brine. The organic layer was separated and washed with brine, dried over MgSO₄ and concentrated in vacuo to afford a pale yellow amorphous (730 mg, 44% purity).

### Reference Example 15

Production of ethyl 2,4(1H,3H)-dioxo-6-(4-methoxyphenyl)-1-(2-methylthiobenzyl)-5-(cyanomethyl)thieno[2,3-d]pyrimidine-3-acetate:

In dimethyl sulfoxide (DMSO) (3 ml) was dissolved the compound obtained in Reference Example 11 (1) (0.67 g, 1.0 mmol) followed by addition of sodium cyanide (50 mg, 1.0 mmol) and the mixture was stirred at 60°C for 6 hours. After cooling, this reaction mixture was poured in iced water (100 ml) and extracted with ethyl acetate (50 ml) and methylene chloride (100 ml, twice). The extracts were pooled, washed with NaCl-water, and dried (MgSO₄) and the solvent was distilled off under reduced pressure. The residue was purified by silica gel column chromatography to provide a light-yellow amorphous solid (0.20 g, 38%).
¹H-NMR (300 MHz, CDCl₃) δ: 1.33 (3H, t, J=7.1 Hz), 2.52 (3H, s), 3.82 (3H, s), 3.94 (2H, s), 4.25 (2H, q, J=7.1 Hz), 4.88 (2H, s), 5.38 (2H, s), 6.95 (2H, d), 7.05 (1H, d), 7.17 (1H, t), 7.34 (2H, d), 7.20-7.40 (2H, m).
IR (KBr): 2978, 2254, 1676, 1607, 1568, 1539, 1483, 1257 cm⁻¹.

The compounds shown in the above Reference Examples are listed in the Table 1.

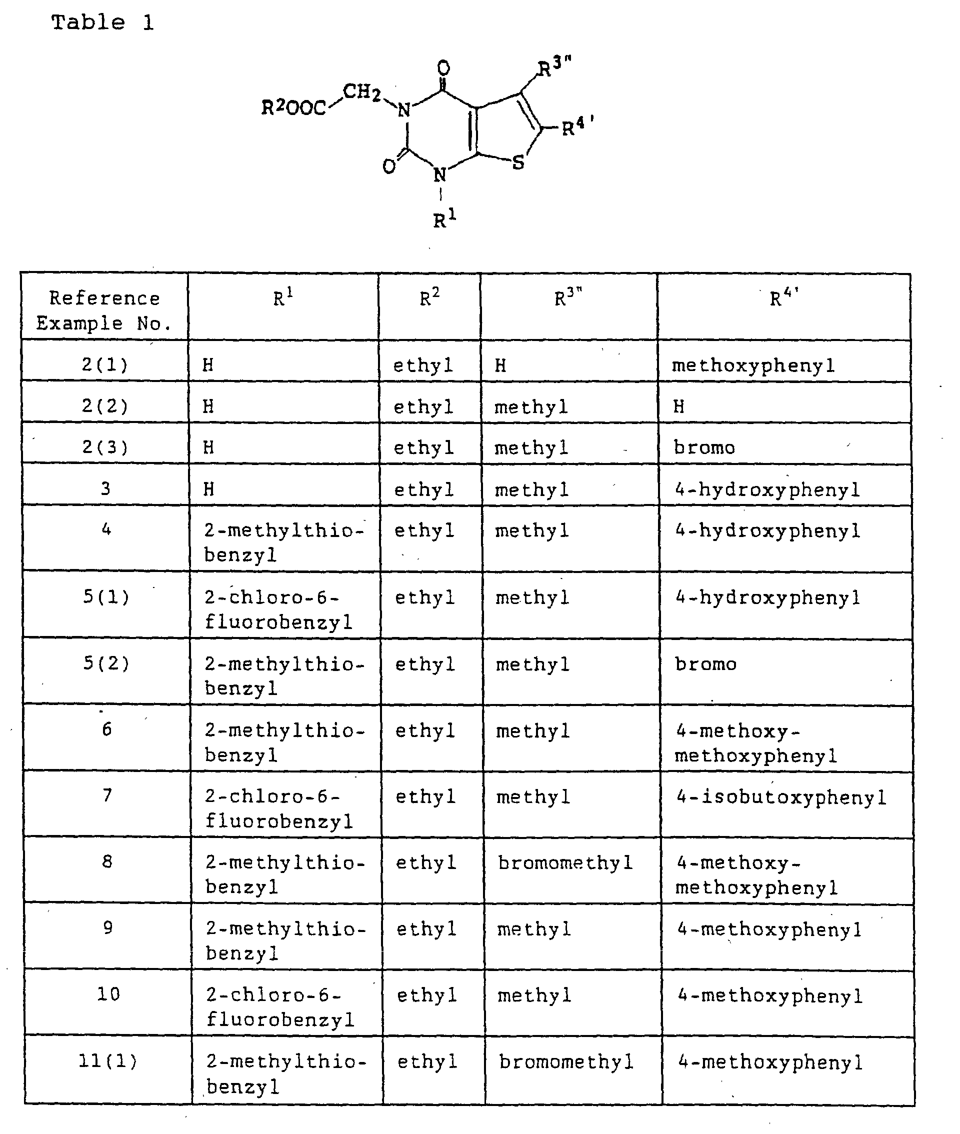

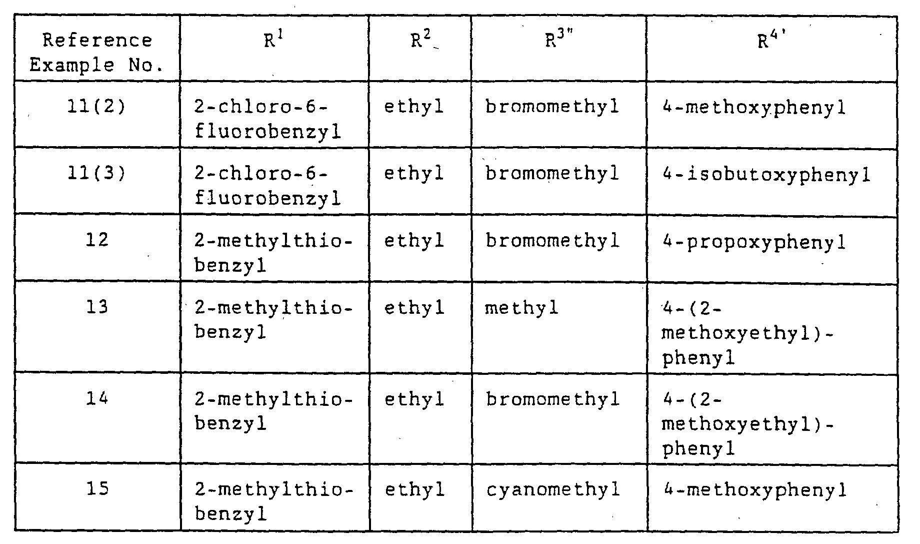

### Example 1

Production of ethyl 2,4(1H,3H)-dioxo-6-(4-methoxy-methoxyphenyl)-1-(2-methylthiobenzyl)-5-(methanesulfonamidomethyl)thieno[2,3-d]pyrimidine-3-acetate:

To a suspension of sodium hydride (60% in oil; 60 mg, 1.5 mmol) in dimethylformamide (10 ml) was added the compound obtained in Reference Example 8 (0.6 g, 1.0 mmol) as well as methanesulfonamide (0.11 g, 1.2 mmol). The mixture was stirred at room temperature for 16 hours, at the end of which time it was concentrated. The residue was distributed between ethyl acetate and aqueous ammonium chloride solution and the aqueous layer was extracted with ethyl acetate. The extracts were combined, washed with NaCl-water, and dried (MgSO₄), and the solvent was distilled off under reduced pressure. The residue was purified by silica gel column chromatography to provide a light-yellow amorphous solid (0.36 g, 59%).
¹H-NMR (300 MHz, CDCl₃) δ: 1.33 (3H, t, J=7.1 Hz), 2.53 (3H, s), 2.88 (3H, s), 3.48 (3H, s), 4.28 (2H, q, J=7.1 Hz), 4.37 (2H, d, J=6.3 Hz), 4.85 (2H, s), 5.19 (2H, s), 5.36 (2H, s), 6.07 (1H, t), 7.0-7.20 (4H, m), 7.25-7.40 (4H, m).

### Example 2

Production of ethyl 2,4(1H,3H)-dioxo-6-(4-methoxy-methoxyphenyl)-1-(2-methylthiobenzyl)-5-(benzenesulfonamidomethyl)thieno[2,3-d]pyrimidine-3-acetate:

The compound obtained in Reference Example 8 (0.6 g) was reacted with benzenesulfonamide in lieu of methanesulfonamide in otherwise the same manner as Example 1 to provide a light-yellow amorphous solid (0.56 g, 83%).
¹H-NMR (300 MHz, CDCl₃) δ: 1.34 (3H, t, J=7.1 Hz), 2.52 (3H, s), 3.50 (3H, s), 4.30 (2H, q, J=7.1 Hz), 4.27 (2H, m), 4.82 (2H, s), 5.21 (2H, s), 5.26 (2H, s), 6.63 (1H, t), 6.97 (1H, d), 7.08 (2H, d), 7.17 (1H, dt), 7.25-7.45 (6H, m), 7.51 (1H, t), 7.66 (2H, dd), 7.94 (1H, d).

### Example 3

Using the compound obtained in Reference Example 8, 11, 12, 13 and 14, the similar procedure as in Example 1 was otherwise repeated to provide the following compounds. Compound 1: Ethyl 2,4(1H,3H)-dioxo-6-(4-methoxyphenyl)-1-(2-methylthiobenzyl)-5-(methanesulfonamidomethyl)thieno[2,3-d]pyrimidine-3-acetate. Yield 91%, amorphous.
Compound 2: Ethyl 2,4(1H,3H)-dioxo-6-(4-methoxyphenyl)-1-(2-chloro-6-fluorobenzyl)-5-(methanesulfonamidomethyl)thieno[2,3-d]pyrimidine-3-acetate. Yield 33%, amorphous.
Compound 3: Ethyl 2,4(1H,3H)-dioxo-6-(4-isobutoxyphenyl)-1-(2-chloro-6-fluorobenzyl)-5-(methanesulfonamidomethyl)thieno[2,3-d]pyrimidine-3-acetate. Yield 29%, amorphous.
Compound 4: Ethyl 2,4(1H,3H)-dioxo-6-(4-propoxyphenyl)-1-(2-methylthiobenzyl)-5-(methanesulfonamidomethyl)thieno[2,3-d]pyrimidine-3-acetate. Yield 85%, amorphous.
Compound 5: Ethyl 2,4(1H,3H)-dioxo-6-(4-methoxymethoxy phenyl)-1-(2-methylthiobenzyl)-5-(ethanesulfonamidomethyl)thieno[2,3-d]pyrimidine-3-acetate. Yield 89%, m.p. 153-155°C.
Compound 6: Ethyl 2,4(1H,3H)-dioxo-6-(4-methoxymethoxy phenyl)-1-(2-methylthiobenzyl)-5-(propanesulfonamidomethyl)thieno[2,3-d]pyrimidine-3-acetate. Yield 85%, m.p. 122-123°C.
Compound 7: Ethyl 2,4(1H,3H)-dioxo-6-(4-propoxyphenyl)-1-(2-methylthiobenzyl)-5-(isopropanesulfonamidomethyl)thieno[2,3-d]pyrimidine-3-acetate. Yield 60%, amorphous.
Compound 8: Ethyl 2,4(1H,3H)-dioxo-6-(4-methoxymethoxy phenyl)-1-(2-methylthiobenzyl)-5-(trifluoromethanesulfonamidomethyl)thieno[2,3-d]pyrimidine-3-acetate. Yield 58%, amorphous.
Compound 9: Ethyl 2,4(1H,3H)-dioxo-6-(4-methoxymethoxy phenyl)-1-(2-methylthiobenzyl)-5-(isopropanesulfonamidomethyl)thieno[2,3-d]pyrimidine-3-acetate. Yield 93%, amorphous.
Compound 10: Ethyl 2,4(1H,3H)-dioxo-6-(4-propoxyphenyl)-1-(2-methylthiobenzyl)-5-(ethanesulfonamidomethyl)thieno[2,3-d]pyrimidine-3-acetate. Yield 84%, m.p. 132-134°C.
Compound 11: Ethyl 2,4(1H,3H)-dioxo-6-(4-(2-methoxyethyl)phenyl)-1-(2-methylthiobenzyl)-5-(methanesulfonamidomethyl)thieno[2,3-d]pyrimidine-3-acetate. Yield 59%, m.p. 131-134°C.

### Example 4

Production of ethyl 2,4(1H,3H)-dioxo-6-(4-methoxyphenyl)-1-(2-methylthiobenzyl)-5-(ethoxycarbonylmethyl)thieno[2,3-d]pyrimidine-3-acetate:

The compound obtained in Reference Example 15 (0.11 g, 0.21 mmol) was dissolved in ethanol (20 ml) followed by addition of saturated HCl-ethanol (10.5 N). (4 ml) and the mixture was refluxed for 48 hours. After cooling, the reaction mixture was distributed between ethyl acetate (50 ml) and saturated NaHCO₃- water (30 ml). The aqueous layer was re-extracted with ethyl acetate (30 ml). The extracts were combined, washed with NaCl-water, and dried (MgSO₄) and the solvent was distilled off under reduced pressure. The residue was crystallized from methanol to provide colorless crystals (0.10 g, 84%). m.p. 117-118°C.

| Elemental analysis for C₂₉H₃₀N₂O₇S•1/2H₂O | | | |
|---|---|---|---|
| | C (%) | H (%) | N (%) |
| Calcd. | 58.87; | 5.28; | 4.73 |
| Found | 58.97; | 5.25; | 4.65 |

¹H-NMR (300 MHz, CDCl₃) δ: 1.27 (3H, t, J=7.1 Hz), 1.29 (3H, t, J=7.1 Hz), 2.53 (3H, s), 3.82 (3H, s), 3.84 (2H, d), 4.19 (2H, q, J=7.0 Hz), 4.22 (2H, q, J=7.1 Hz), 4.82 (2H, s), 5.34 (2H, s), 6.89 (2H, d), 7.05 (1H, d), 7.15 (1H, t), 7.25 (2H, d), 7.32 (2H, t).

### Example 5

Production of 2,4(1H,3H)-dioxo-6-(4-methoxymethoxyphenyl)-1-(2-methylthiobenzyl)-5-(methanesulfonamidomethyl)thieno[2,3-d]pyrimidine-3-aretic acid:

The compound obtained in Example 1 (0.5 g, 0.83 mmol) was dissolved in tetrahydrofuran (10 ml)-methanol (2 ml) followed by addition of 1N-sodium hydroxide-water (2 ml). This mixture was stirred at room temperature for 4 hours, after which 1N hydrochloric acid solution (2 ml) was added. The mixture was then concentrated and the residue was distributed between ethyl acetate and aqueous ammonium chloride solution. The aqueous layer was extracted with ethyl acetate. The extracts were combined, washed with NaCl-water, and dried (MgSO₄) and the solvent was distilled off under reduced pressure. The residue was purified by silica gel column chromatography to give a light-yellow solid (0.55 g). This product was recrystallized from ethyl acetate-isopropyl ether to provide light-yellow crystals (0.40 g, 76%). m.p. 208-209°C.

| Elemental analysis for C₂₆H₂₇N₃O₈S₃·1/2H₂O | | | |
|---|---|---|---|
| | C (%) | H (%) | N (%) |
| Calcd. | 50.80; | 4.59; | 6.83 |
| Found | 50.75; | 4.53; | 6.79 |

¹H-NMR (300 MHz, DMSO) δ: 2.55 (3H, s), 2.87 (3H, s), 3.41 (3H, s), 4.30 (2H, s), 4.41 (2H, s), 5.21 (2H, s), 5.24 (2H, s), 6.91 (1H, t), 7.02 (1H, d), 7.11 (2H, d), 7.15 (1H, d), 7.33 (1H, t), 7.42 (2H, d).

### Example 6

Production of ethyl 2,4(1H,3H)-dioxo-6-(4-isobutoxyphenyl)-1-(2-methylthiobenzyl)-5-(methanesulfonamidomethyl)thieno[2,3-d]pyrimidine-3-acetate:

To a solution of ethyl 2,4(1H,3H)-dioxo-6-(4-hydroxyphenyl)-1-(2-methylthiobenzyl)-5-(methanesulfonamidomethyl)thieno[2,3-d]pyrimidine-3-acetate (0.30 g), which was synthesized from ethyl 2,4(1H,3H)-dioxo-6-(4-methoxymethoxyphenyl)-1-(2-methylthiobenzyl)-5-(methanesulfonamidomethyl)thieno [2,3-d]pyrimidine-3-acetate (which was obtained in Example 1) with 1N hydrochloric acid in tetrahydrofuran at room temperature for 3 hours, in dimethylformamide (DMF) (25 ml) was added isobutyliodide (0.30 g) and K₂CO₃ (0.3 g). The mixture was stirred at room temperature for 24 hours. Then the mixture was evaporated in vacuo to give the residue, which was partitioned between ethyl acetate (50 ml) and aq.NH₄Cl (30 ml). The organic solution was dried with Na₂SO₄ and evaporated in vacuo to give a yellow amorphous, which was chromatographed on silica gel to provide a yellow amorphous (0.11 g, 33%).

### Example 7

Using the compounds obtained in Example 1, the similar procedure as in Example 6 is repeated to provide the following compounds:
Compound 1: Ethyl 2,4(1H,3H)-dioxo-6-(4-carboxymethoxyphenyl)-1-(2-methylthiobenzyl)-5-(methanesulfonamidomethyl)thieno[2,3-d]pyrimidine-3-acetate. Yield 70%, amorphous.
Compound 2: Ethyl 2,4(1H,3H)-dioxo-6-(4-allyloxyphenyl)-1-(2-methylthiobenzyl)-5-(methanesulfonamidomethyl)thieno[2,3-d]pyrimidine-3-acetate. Yield 84%, amorphous.
Compound 3: Ethyl 2,4(1H,3H)-dioxo-6-(4-butoxyphenyl)-1-(2-methylthiobenzyl)-5-(methanesulfonamidomethyl)thieno[2,3-d]pyrimidine-3-acetate. Yield 82%, amorphous.
Compound 4: Ethyl 2,4(1H,3H)-dioxo-5-[4-(2,2,2-trifluoroethoxyphanyl)]-1-(2-methylthiobenzyl)-5-(methanesulfonamidomethyl)thieno(2,3-d)pyrimidine-3-acetate.

### Example 8

Production of ethyl 2,4(1H,3H)-dioxo-6-(4-methylamino carbonylmethoxyphenyl)-1-(2-methylthiobenzyl)-5-(methanesulfonamidomethyl)thieno [2,3-d]pyrimidine-3-acetate:

The compound 1 obtained in Example 7 was reacted with isobutylchloroformate and triethylamine in tetrahydrofuran (THF) at 0°C for three hours to provide acid anhydride compound, which was converted to amide derivative with methylamine. Yield 100%, amorphous.

### Example 9

Using the compounds obtained in Example 7, the procedure as in Example 8 was repeated to produce the following compounds:
Compound 1: Ethyl 2,4(1H,3H)-dioxo-6-(4-propylaminocarbonylmethoxyphenyl)-1-(2-methylthiobenzyl)-5-(methanesulfonamidomethyl)thieno [2,3-d]pyrimidine-3-acetate. Yield 95%, amorphous.
Compound 2: Ethyl 2,4(1H,3H)-dioxo-6-(4-piperazinecarbonylmethoxyphenyl)-1-(2-methylthiobenzyl)-5-(methanesulfonamidomethyl)thieno [2,3-d]pyrimidine-3-acetate. Yield 66%, amorphous.

### Example 10

(1) Production of pivaloyloxymethyl 2,4(1H,3H)-dioxo-6-(4-methoxymethoxyphenyl)-1-(2-methylthiobenzyl)-5-(methanesulfonamidomethyl)-thieno[2,3-d]pyrimidine-3-acetate:

To an ice-cooled mixture of 2,4(1H,3H)-dioxo-5-methanesulfonamidomethyl-6-(4-methoxymethoxyphenyl)-1-(2-methylthiobenzyl)thieno[2,3-d]pyrimidine-3-acetic acid obtained in Example 5 (0.25 g, 0.413 mmol), K₂CO₃ (86 mg, 0.622 mmol) and KI (83 mg, 0.50 mmol) in DMF (8 ml) was added dropwise chloromethyl pivalate (72 ml, 0.50 mmol). After being stirred at 0°C to room temperature for 22 hours, the mixture was concentrated in vacuo and the residue was partitioned between ethyl acetate and brine. The aqueous phase was separated and extracted with ethyl acetate. The combined extracts were washed with brine, dried over MgSO₄, and concentrated in vacuo. The residue was subjected to silica gel column chromatography by eluting with ethyl acetate - hexane (4:6 - 1:1) to give the product (0.24 g, 80.8%) as a colorless syrup.

Crystallization from ethyl acetate-ether-hexane afforded the product (0.203 g, 72.6%) as white crystals. Yield 81%, m.p. 74-77°C.

(2) Employing the compound produced in Example 5 as the starting material, in accordance with substantially the same procedure as described the above item (1) of Example 10, the following compound is produced. (R.S)-1-(cyclohexyloxycarbonyloxy)ethyl 2,4(1H,3H)-dioxo-6-(4-methoxymethoxyphenyl)-1-(2-methylthiobenzyl)-5-(methanesulfonamidomethyl)thieno [2,3-d]pyrimidine-3-acetate.

### Example 11

Using the compounds obtained in Examples 2, 3, 4, 6, 7, 8 or 9, the procedure of Example 5 is otherwise repeated to provide the following compounds.
Compound 1: 2,4(1H,3H)-Dioxo-6-(4-methoxymethoxyphenyl)-1-(2-methylthiobenzyl)-5-(benzenesulfonamidomethyl)thieno[2,3-d]pyrimidine-3-acetic acid. Yield 68%, m.p. 120-125°C.
Compound 2: 2,4(1H,3H)-Dioxo-6-(4-methoxyphenyl)-1-(2-methylthiobenzyl)-5-(methanesulfonamidomethyl)-thieno[2,3-d]pyrimidine-3-acetic acid. Yield 76%, m.p. 208-209°C.
Compound 3: Ethyl 2,4(1H,3H)-dioxo-6-(4-methoxyphenyl)-1-(2-methylthiobenzyl)-5-(carboxylmethyl)thieno[2,3-d]-pyrimidine-3-acetate. Yield 65%, m.p. 243-245°C.
Compound 4: 2,4(1H,3H)-Dioxo-6-(4-methoxyphenyl)-1-(2-chloro-6-fluorobenzyl)-5-(methanesulfonamidomethyl)-thieno[2,3-d]pyrimidine-3-acetic acid. Yield 57%, amorphous.
Compound 5: 2,4(1H,3H)-Dioxo-6-(4-isobutoxyphenyl)-1-(2-chloro-6-fluorobenzyl)-5-(methanesulfonamidomethyl)-thieno[2,3-d]pyrimidine-3-acetic acid. Yield 30%, m.p. amorphous.
Compound 6: 2,4(1H,3H)-Dioxo-6-(4-isobutoxyphenyl)-1-(2-methylthiobenzyl)-5-(methanesulfonamidomethyl)-thieno[2,3-d]pyrimidine-3-acetic acid.
Compound 7: 2,4(1H,3H)-Dioxo-6-(4-propoxyphenyl)-1-(2-methylthiobenzyl)-5-(methanesulfonamidomethyl)-thieno[2,3-d]pyrimidine-3-acetic acid. Yield 84%, amorphous.
Compound 8: 2,4(1H,3H)-Dioxo-6-(4-butoxyphenyl)-1-(2-methylthiobenzyl)-5-(methanesulfonamidomethyl)-thieno[2,3-d]pyrimidine-3-acetic acid. Yield 85%, amorphous.
Compound 9: 2,4(1H,3H)-Dioxo-6-(4-propoxyphenyl)-1-(2-methylthiobenzyl)-5-(ethanesulfonamidomethyl)-thieno[2,3-d]pyrimidine-3-acetic acid. amorphous.
Compound 10: 2,4(1H,3H)-Dioxo-6-(4-(2-methoxyethyl)phenyl)-1-(2-methylthiobenzyl)-5-(methanesulfonamidomethyl)thieno[2,3-d]pyrimidine-3-acetic acid. Yield 73%, m.p. 167-168°C.
Compound 11: 2,4(1H,3H)-Dioxo-6-(4-methoxymethoxy phenyl)-1-(2-methylthiobenzyl)-5-(isopropanesulfonamidomethyl)thieno[2,3-d]pyrimidine-3-acetic acid. Yield 64%, m.p. 112-114°C.
Compound 12: 2,4(1H,3H)-Dioxo-6-(4-methylaminocarbonylmethoxyphenyl)-1-(2-methylthiobenzyl)-5-(methanesulfonamidomethyl)thieno[2,3-d]pyrimidine-3-acetic acid. Yield 58%, amorphous.
Compound 13: 2,4(1H,3H)-Dioxo-6-(4-propylamino carbonylmethoxyphenyl)-1-(2-methylthiobenzyl)-5-(methanesulfonamidomethyl)thieno[2,3-d]pyrimidine-3-acetic acid. Yield 81%, amorphous.
Compound 14: 2,4(1H,3H)-Dioxo-6-(4-piperazine carbonylmethoxyphenyl)-1-(2-methylthiobenzyl)-5-(methanesulfonamidomethyl)thieno[2,3-d]pyrimidine-3-acetic acid. Yield 84%, amorphous.
Compound 15: 2,4(1H,3H)-Dioxo-6-(4-propoxyphenyl)-1-(2-methylthiobenzyl)-5-(isopropanesulfonamidomethyl)-thieno[2,3-d]pyrimidine-3-acetic acid. Yield 84%, amorphous.
Compound 16: 2,4(1H,3H)-Dioxo-6-(4-methoxymethoxyphenyl)-1-(2-methylthiobenzyl)-5-(ethanesulfonamidomethyl)thieno[2,3-d]pyrimidine-3-acetic acid. Yield 80%, m.p. 125-128°C

| Elemental analysis for C₂₇H₂₉N₃O₈S₃•1.0H₂O | | | |
|---|---|---|---|
| | C (%) | H (%) | N (%) |
| Calcd. | 50.85; | 4.90; | 6.59 |
| Found | 51.15; | 4.78; | 6.54 |

¹H-NMR (300 MHz, CDCl₃) δ: 1.33 (3H, t, J=7.4 Hz), 2.53 (3H, s), 2.96 (2H, q, J=7.4 Hz), 3.48 (3H, s), 4.35 (2H, d, J=6.6Hz), 4.92 (2H, s), 5.19 (2H, s), 5.36 (2H, s), 6.05 (1H, t, J=6.6 Hz), 7.01-7.37 (8H, m).
IR (KBr): 1702, 1649, 1543, 1487 cm⁻¹
Mass spectrum: 620.1 (MH⁺)
Compound 17: Ethyl 2,4(1H,3H)-dioxo-6-(4-methoxymethoxyphenyl)-1-(2-methylthiobenzyl)-5-(propanesulfonamidomethyl)thieno[2,3-d]pyrimidine-3-acetic acid. Yield 93%, m.p. 123-124°C
Compound 18: Ethyl 2,4(1H,3H)-dioxo-6-(4-methoxymethoxyphenyl)-1-(2-methylthiobenzyl)-5-(trifluoromethanesulfonamidomethyl)thieno[2,3-d]pyrimidine-3-acetic acid. Yield 52%, amorphous.
Compound 19: 2,4(1H,3H)-Dioxo-6-[4-(2,2,2-trifluoroethoxyphenyl)]-1-(2-methylthiobenzyl)-5-(methanesulfonamidomethyl)thieno[2,3-d]pyrimidine-3-acetic acid.

The compounds shown in the above Examples are listed in the Table 2.

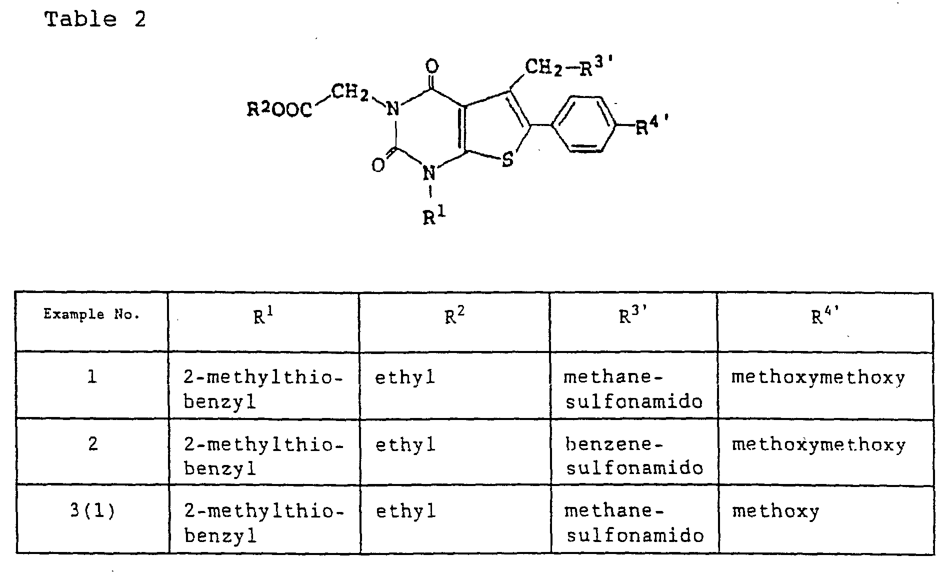

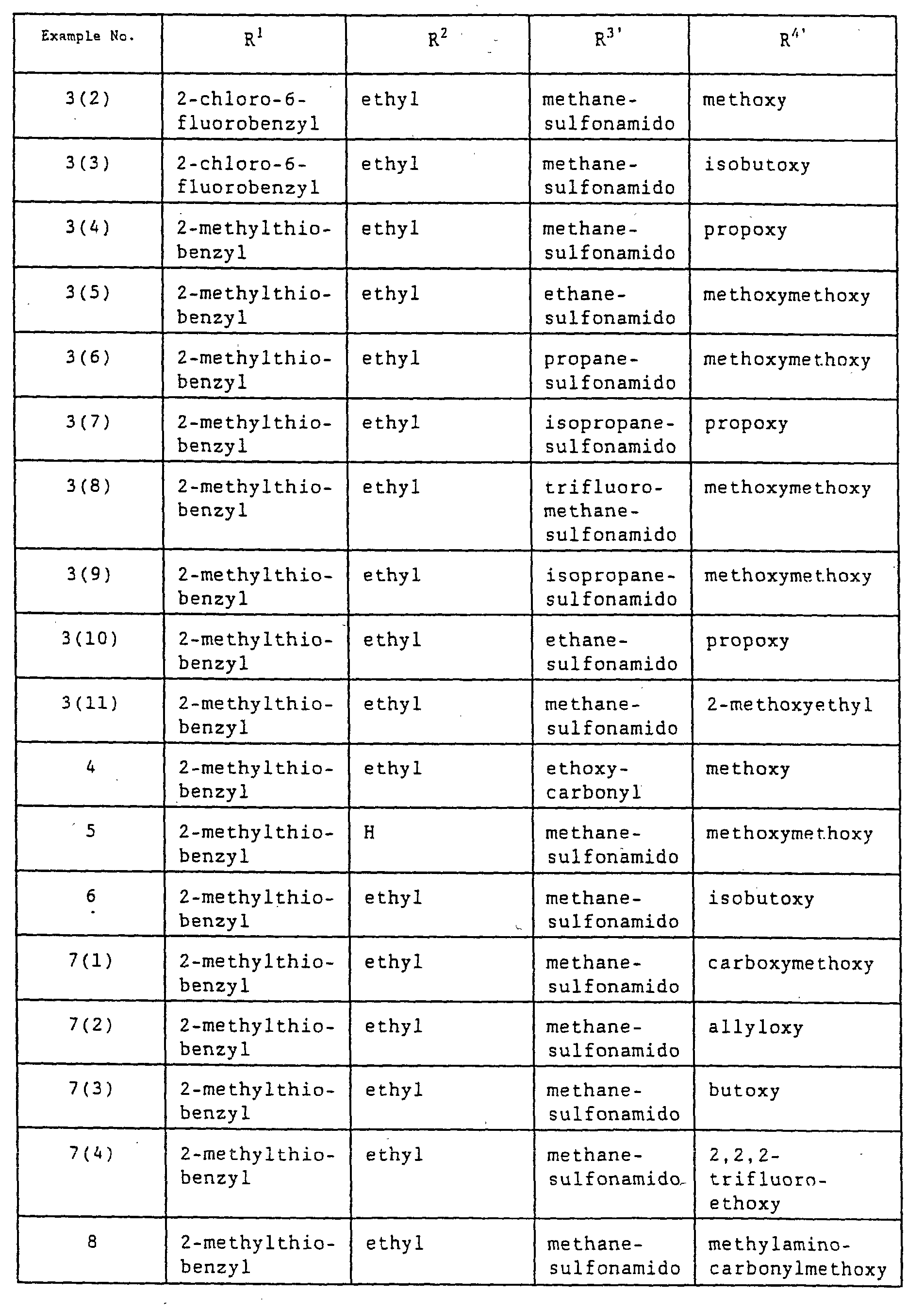

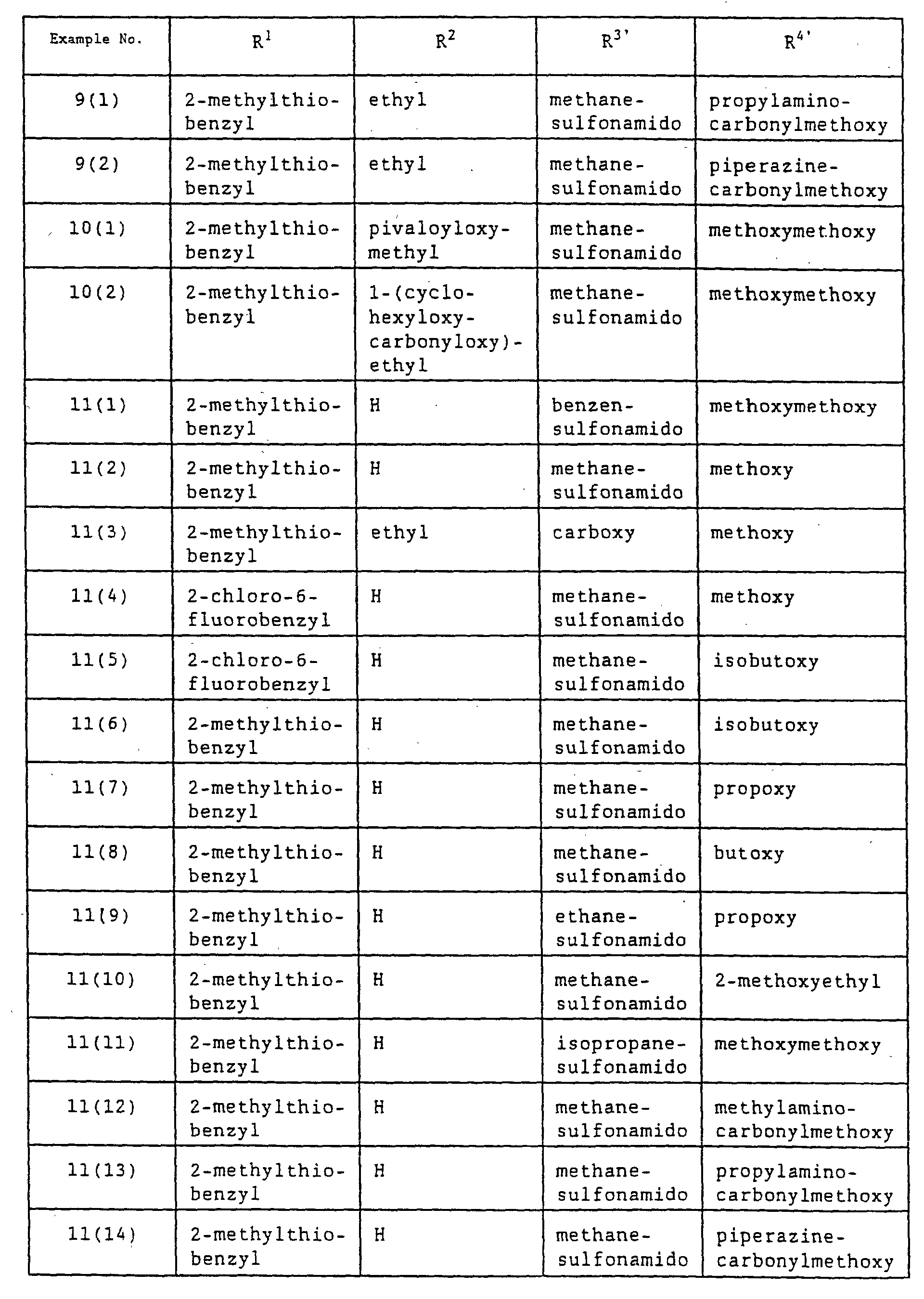

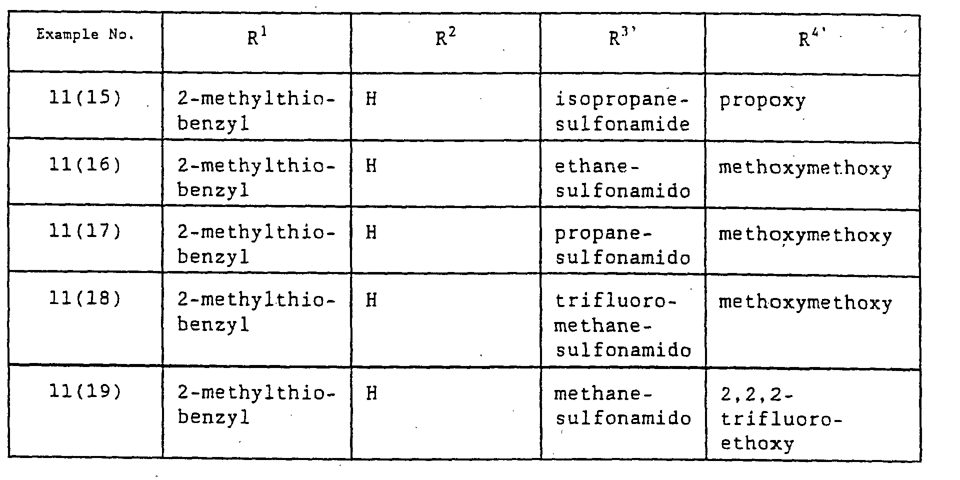

### Example 12

A tablet is prepared by a conventional method using 100 mg of the compound produced in Example 1, 165 mg of lactose, 25 mg corn starch, 4 mg of polyvinyl alcohol and 1 mg of magnesium stearate.

### Example 13

The compound (5 g) produced in Example 1 is dissolved in a distilled water for injection to make the total volume 100 ml. The solution is subjected to an aseptic filtration using a membrane filter of 0.22 micrometer (manufactured by Sumitomo Electric, Japan or by Saltorius, Germany). Each 2 ml of the filtrate is placed in a washed and sterilized vial and dried by freezing by a conventional method to prepare a freeze-dried injection of 100 mg/vial.

### Example 14

A tablet is prepared by a conventional method using 100 mg of the compound produced in Example 5, 165 mg of lactose, 25 mg of corn starch, 4 mg of polyvinyl alcohol and 1 mg of magnesium stearate.

### Example 15

The compound (5 g) produced in Example 5 is dissolved in a distilled water for injection to make the total volume 100 ml. The solution is subjected to an aseptic filtration using a membrane filter of 0.22 micrometer (manufactured by Sumitomo Electric, Japan or by Saltorius, Germany). Each 2 ml of the filtrate is placed in a washed and sterilized vial and dried by freezing by a conventional method to prepare a freeze-dried injection of 100 mg/vial.

### Example 16

A tablet is prepared by a conventional method using 100 mg of the compound (5) produced in Example 3, 165 mg of lactose, 25 mg of corn starch, 4 mg of polyvinyl alcohol and 1 mg of magnesium stearate.

### Example 17

The compound (5) (5 g) produced in Example 3 was dissolved in a distilled water for injection to make the total volume 100 ml. The solution was subjected to an aseptic filtration using a membrane filter 0f 0.22 micrometer (manufactured by Sumitomo Electric, Japan or by Saltorius, Germany). Each 2 ml of the viltrate was placed in a washed and sterilized vial and dried by freezing to prepare a freeze-dried injection of 100 mg/vial.

### Example 18

A tablet is prepared by a conventional method using 100 mg of the compound (16) produced in Example 11, 165 mg of lactose, 25 mg of corn starch, 4 mg of polyvinyl alcohol and 1 mg of magnesium stearate.

### Example 19

The compound (16) (5 g) produced in Example 11 is dissolved in a distilled water for injection to make the total volume 100 ml. The solution is subjected to an aseptic filtration using a membrane filter of 0.22 micrometer (manufactured by Sumitomo Electric, Japan or by Saltorius, Germany). Each 2 ml of the filtrate is placed in a washed and sterilized vial and dried by freezing by a conventional method to prepare a freeze-dried injection of 100 mg/vial.

### Experimental Example 1

Binding Test to ET_{A} receptor expressed in CHO cell:

### Procedure

- cells:: CHO cell expressing human ET_{A} 24 endothelin receptor, i.e. ET_{A} 24 cells
- medium:: DMEM 10% FCS Gln, nonessential amino acids, penicillin, streptomycin

Cells were seeded in 12 wells of 24 well plates at a density of 2x10⁵ cells/well (1 ml medium/well). On the next day, [³H]arachidonic acid was added to each well to be 250 nCi(nanocurie)/ml. On the next day, the medium was sucked from the wells by the use of an aspirator to remove free arachidonic acid and floating cells, and then 0.5 ml of medium was added. This procedure was repeated again. After allowing to stand for 30 minutes in a CO₂ incubator, the medium was exchanged rapidly.

The sample of compound obtained in Example 5 or compound 16 of Example 11 was stepwise diluted with a buffer solution for dilution, containing 3.15x10⁻⁸M endothelin-1 (ET-1) {20 mM Tris, 5 mM Mg(AcO)₂, 2 mM EGTA, 0.03% NaN₃, 0.1% BSA, 0.05% CHAPS}. 10 µl of the solution was added to each well (final concentration of ET-1: 6.3x10⁻¹⁰M). The maximum reaction value was estimated by adding 10 µl of 3.15x10⁻⁸M ET-1. The radio activity under no stimulation was estimated by adding the buffer solution for dilution. After allowing to-stand for 30 minutes in the CO₂ incubator, the medium was completely collected and the radio activity of [³H]arachidonic acid released in the medium was measured by a liquid scintillation counter. IC₅₀ values were calculated by hill plot from the concentration and relative reaction value of each sample.
Abbreviations:
DMEM: Dulbecco's medified Eagle Medium
FCS : fetal calf serum
AcO : acetyloxy
EGTA: ethyleneglycol bis(2-aminoethylether)tetraacetic acid
BSA : bovine serum albumin
CHAPS: 3-[(3-chloroamidopropyl)dimethylammonio]-1-propanesulfonate

### Results

IC₅₀ values obtained are shown in Table 3:

**[Table 3]**

| Test compound | IC₅₀ value: µM |
|---|---|
| Compound obtained in Example 5 | 0.39 (n=2) |
| | |
| Compound 16 of Example 11 | 0.11 (n=2) |

### Experimental Example 2

Binding Test to ET_{B} receptor expressed in CHO cell:

### Procedure

- cells:: CHO cell expressing human ET_{B} endothelin receptor, i.e. ET_{B} 12 cells
- medium:: DMEM 10% FCS Gln, nonessential amino acids, penicillin, streptomycin

Cells were seeded in 12 wells of 24 well plates at a density of 2x10⁵ cells/well (1 ml medium/well). On the next day, [³H]arachidonic acid was added to each well to be 250 nCi(nanocurie)/ml. On the next day, the medium was sucked from the wells by the use of an aspirator to remove free arachidonic acid and floating cells, and then 0.5 ml of medium was added. This procedure was repeated agian. After allowing to stand for 30minutes in a CO₂ incubator, the medium was exchanged rapidly.

The sample of compound obtained in Example 5 or compound 16 of Example 11 was stepwise diluted with a buffer solution for dilution, containing 3.15x10⁻⁸M endothelin-1 (ET-1) {20 mM Tris, 5mM Mg(AcO)₂, 2 mM EGTA, 0.03% NaN₃, 0.1% BSA, 0.05% CHAPS}. 10 µl of the solution was added to each well (final concentration of ET-1: 6.3x10⁻¹⁰M). The maximum reaction value was estimated by adding 10 µl of 3.15x10⁻⁸M ET-1. The radio activity under no stimulation was estimated by adding the buffer solution for dilution. After allowing to-stand for 30 minutes in the CO₂ incubator, the medium was completely collected and the radio activity of [³H]arachidonic acid released in the medium was measured by a liquid scintillation counter. IC₅₀ values were calculated by Hill Plot from the concentration and relative reaction value of each sample.

### Results

IC₅₀ values obtained are shown in Table 4:

**[Table 4]**

| Test compound | IC₅₀ value: µM |
|---|---|
| Compound obtained in Example 5 | 0.49 (n=2) |
| | |
| Compound 16 of Example 11 | 0.13 (n=2) |

### Experimental Example 3

Inhibition Test on coronary artery where ET_{A} is expressed:

### Procedure

3-mm ring samples for vehicle group and drug-treating group were prepared by removing fat and connective tissue from coronary artery enucleated from porcine heart and obtained from the adjacent portions thereof. The samples, hanging in Magnus tube filled with Krebs solution, were stabilized for 90 minutes under 2 g of static tension. After subjecting the samples to constriction for 10 minutes by potassium chloride (KCl) (60 mM) to obtain the maximum reaction, the samples were then washed and stabilized for 60 minutes. After pre-treating compound obtained in Example 5 or Compound 16 of Example 11 or vehicle (H₂O) for 30 minutes, ET-1 (2 mM) was added to observe the maximum constriction.

The constriction efficiency (% KCl) of ET-1 was calculated as a relative value to KCl constriction of each sample which was regarded as 100%. Further, the inhibiting efficiency was calculated from the constriction efficiency of the drug-treating group calculated as a relative value to the constriction of the vehicle group which was regarded as 100%.

### Results

The results are shown in Table 5.

**[Table 5]**

| | % inhibition (MEAN ± S.E.M.) (n=) artery (ET-1 3nM) | | Binding IC₅₀ (µM) |
|---|---|---|---|
| Compound | 0.1 µM | 1 µM | ET_{A} |
| Compound in Example 5 | 7.6 ± 37.3 (3) | 78.1 ± 11.9 (4) | 0.0076 |
| Compound 16 of Example 11 | - | 33.1 ± 8.6 (4) | 0.0061 |

It is apparent from the results of Table 5 that in the ring samples of porcine coronary artery in which ET_{A} is expressed, the compounds of present invention suppress vascular (smooth muscle) constriction through the agonist of ET_{A}, i.e. ET-1 (3 nM).

Thus, it was confirmed that the compounds of present invention are antagonists for ET_{A} receptor.

### Experimental Example 4

Inhibition Test on coronary vein where ET_{B} is expressed:

### Procedure 1

3-mm ring samples for vehicle group and drug-treating group were prepared by removing fat and connective tissue from coronary vein enucleated from porcine heart and obtained from the adjacent portions thereof. The samples, hanging in Magnus tube filled with Krebs solution, were stabilized for 90 minutes under 0.5 g static tension. After subjecting the samples to constriction for 10 minutes by potassium chloride (KCl) (60 mM) to obtain the maximum reaction, the samples were washed and stabilized for 60 minutes. After pre-treating compound obtained in Example 5 or Compound 16 of Example 11 or vehicle (H₂O) for 30 minutes, S6c (1 nM) (S6c: sarafotoxin S6c, peptide type snake toxin consisting of 21 amino acids, it is useful for the selective agonist to ET_{B} receptor owing to the similarity of its structure to endothelin) was added to observe the maximum constriction.

The constriction efficiency (% KCl) of S6c was calculated as a relative value to KCl constriction of each sample which was regarded as 100%. Further, the inhibiting efficiency was calculated from the constriction efficiency of the drug-treating group calculated as a relative value to the constriction of the vehicle group which was regarded as 100%.

### Results

The results are shown in Table 6.

**[Table 6]**

| | % inhibition (MEAN ± S.E.M.) (n=) vein (S6c 1 nM) | | Binding IC₅₀ (µM) |
|---|---|---|---|
| Compound | 0.1 µM | 10 µM | ET_{B} |
| Compound in Example 5 | 73.4 ± 3.1 (3) | 100 ± 0 (4) | 0.100 |
| Compound 16 of Example 11 | 53.3 ± 4.4 (4) | 98.0 ± 1.1 (4) | 0.054 |

It is apparent from the results of Table 6 that in the ring samples of porcine coronary vein in which ET_{B} is expressed, the compounds of present invention suppress vascular (smooth muscle) constriction through the agonist of ET_{B}, i.e. S6c (1 nM).

Thus, it was confirmed that the compounds of present invention are antagonists for ET_{B} receptor.

### Experimental Example 5

Binding Test to ET_{A} receptor expressed in an insect cell Sf9:

### Procedure

Endothelin (ET) receptors were prepared by diluting fractions of insect cell (Sf9) membrane having human endothelin-A (ETA) receptors expressed, with an assay buffer {20 mM Tris-HCl, 2 mM EGTA (ethyleneglycol bls(2-aminoethylether) tetra acetic acid), 5 mM magnesium acetate, 0.1% BSA (bovine serum albumin), 0.03% NaN₃, 0.5 mM PMSF (phenyl methyl sulfonyl fluoride), 20 µg/ml leupeptin, 4 µg/ml E-64 (products of the Peptide Institute, Japan), 1 µg/ml pepstatin, (pH 7.2)} respectively in a concentration of 1.4 µg/ml in the former case and 0.7 µg/ml in the latte case.

To 100 µl of each portion was added 5 nM[¹²⁵I] endothelin-1 (2 µl). A dimethylsulfoxide solution (3 µl) of the test compound was added thereto and incubated at 25°C for 60 minutes.

And, to determine the maximum binding amount (B₀) and non-specific binding amount (NSB), lots to which a dimethyl sulfoxide solution (3 µl) or a dimethyl sulfoxide solution (3 µl) containing endothelin-1 (10⁻³M) had been added, were also incubated.

These lots were supplemented with 0.05% CHAPS(3-[(3-chloroamidopropyl)dimethylammonio]-1-propanesulfonate)-assay buffer (1.5 ml), subjected to filtration through a glass fiber filter GF/F (trade name; product of Whatman Ltd. (England)), and then washed with the same buffer (1.5 ml)).

Radioactivity on the filter was counted in a gamma-counter to determine the Percent Maximum Binding (PMB) in accordance with the aforesaid calculation formula. The concentration causing PMB=50% was determined as IC₅₀ value. IC₅₀ values of some of the compounds of this invention, synthesized in the above-mentioned examples, are shown in Table 7.

**Table 7**

| Test compound (Compounds are shown by the Example No.) | IC₅₀ value: µM Human endotherin-A receptor |
|---|---|
| 5 | 0.011 |
| potassium salt of 5 | 0.0076 |
| 11(7) | 0.018 |
| 11(9) | 0.015 |
| 11(11) | 0.0066 |
| 11(15) | 0.011 |
| 11(16) | 0.0061 |
| 11(17) | 0.022 |

According to the result shown in the Table 5, it has been proved that the compound or its salt of the present invention have excellent endothelin receptor antagonistic action to endothelin-B receptor.

### Experimental Example 6

Binding Test to ET_{B} receptor expressed in an insect cell Sf9:

### Procedure

Endothelin (ET) receptors were prepared by diluting fractions of insect cell (Sf9) membrane having human endothelin-B (ETB) receptors expressed, with an assay buffer {200 mM Tris-HCl, 2 mM EGTA (ethylenegiycol bis(2-aminoethylether) tetra acetic acid), 5 mM magnesium acetate, 0.1% BSA (bovine serum albumin), 0.03% NaN₃, 0.5 mM PMSF(phenyl methyl sulfonyl fluoride), 20 µg/ml leupeptin, 4 µg/ml E-64 (products of the Peptide Institute), 1 µg/ml pepstatin, (pH 7.2)} respectively in a concentration of 1.4 µg/ml in the former case and 0.7 µg/ml in the latter case.

To 100 µl of each portion was added 5 nM[¹²⁵I] endothelin-1 (2 µl). A dimethylsulfoxide solution (3 µl) of the sample was added thereto and incubated at 25°C fpr 60 minutes.

And, to determine the maximum binding amount (B₀) and non-specific binding amount (NSB), lots to which a dimethyl sulfoxide solution (3 µl) or a dimethyl sulfoxide solution (3 µl) containing endothelin-1 (10⁻⁵M) had been added, were also incubated.

These lots were supplemented with 0.05% CHAPS(3-[(3-chloroamidopropyl)dimethylammonio]-1-propanesulfonate)-assay buffer (1.5 ml), subjected to filtration through a glass fiber filter GF/F (trade name; product of Whatman Ltd. (England)), and then washed with the same buffer (1.5 ml).

Radioactivity on the filter was counted in a gamma-counter to determine the Percent Maximum Binding (PMB) in accordance with the aforesaid calculation formula. The concentration causing PMB=50% was determined as IC₅₀ value. IC₅₀ values of some of the compounds of this invention, synthesized in the above-mentioned examples, are shown in Table 8.

**Table 8**

| Test compound (Compounds are shown by the Example No.) | IC₅₀ value: µM Human endotherin-B receptor |
|---|---|
| 5 | 0.20 |
| potassium salt of 5 | 0.10 |
| 11(7) | 0.22 |
| 11(9) | 0.11 |
| 11(11) | 0.090 |
| 11(15) | 0.094 |
| 11(16) | 0.054 |
| 11(17) | 0.047 |

According to the result shown in the Table 6, it has been proved that the compound or its salt of the present invention have excellent endothelin receptor antagonistic action to endothelin-A receptor.

The potassium salt of the compound of the Working Example 5 was produced by employing the compound of the Working Example 5, potassium carbonate and water-ethanol in a conventional manner.

### Industrial Applicability

The thienopyrimidine derivative of the present invention possesses outstanding endothelin receptor antagonist activity and, therefore, the endothelin antagonist composition containing this thienopyrimidine derivative in accordance with the invention can be used with advantage as a prophylactic or therapeutic drug for acute renal failure, myocardial infarction, liver disorder, angina pectoris, cerebral infarction, cerebrovasospasm, hypertension, kidney disease, asthma, ectopic angina, Rayneau syndrome, pulmonary hypertension, surgical shock, chronic heart failure, atherosclerosis, cardiac hypertrophy, migrane, etc., as a prophylactic or therapeutic drug for organ surgery- or graft-associated hypofunction of organs, or as a prophylactic drug for vascular restenosis following percutaneous transluminal coronary angioplasty (PTCA), or as an inhibitor for vasoconstriction of coronary artery, coronary vein, cerebrovascular system or pulmonary vascular system.

## Claims

1. A compound of the formula: 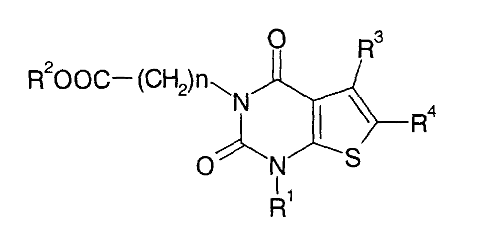 wherein each of R¹ and R² are (a) hydrogen, or (b) a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-membered nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxycarbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) a group of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido;
R³ is a C₁₋₆ alkyl group substituted by a C₁₋₆ alkoxycarbonyl or a group of the formula: -NH-SO₂-R⁵ wherein R⁵ is (1) C₁₋₆ alkyl which may be substituted by halogen, or (2) C₆₋₁₄ aryl;
R⁴ is a phenyl group which may be substituted by (1) C₁₋₄ alkoxy which may be substituted by C₁₋₆ alkoxy, carboxy, C₁₋₆ alkyl-carbamoyl, piperazinecarbonyl or halogen, (2) C₇₋₈ aralkyloxy, (3) C₁₋₄ alkyl which may be substituted by hydroxy, oxo or C₁₋₃ alkoxy, (4) C₁₋₆ alkanoyl, (5) C₂₋₄ alkenyloxy, (6) C₁₋₆ alkoxy-carbonyl or (7) C₁₋₆ alkyl-carbamoyl; and
n is an integer of 1 to 3,
or a salt thereof.

2. A compound according to Claim 1, wherein R¹ is a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-memberd nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) a group of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido.

3. A compound according to Claim 1, wherein R¹ is C₇₋₂₀ aralkyl group which may be substituted by halogen, nitro, hydroxy, cyano, C₁₋₄ alkyl, C₁₋₄ alkylthio or C₁₋₄ alkoxy.

4. A compound according to Claim 1, wherein R¹ is benzyl which may be substituted by halogen or C₁₋₄ alkylthio.

5. A compound according to Claim 1, wherein R² is a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-memberd nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) a group of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido.

6. A compound according to Claim 1, wherein R² is a C₁₋₁₀ alkyl group which may be substituted by 1 to 6 substituents selected from the group consisting of (1) halogen, (2) nitro, (3) nitroso, (4) cyano, (5) hydroxy which may be substituted by (i) C₁₋₆ alkyl which may be substituted by hydroxy, C₁₋₆ alkoxy, C₁₋₃ alkoxy-C₁₋₃ alkoxy, C₁₋₃ alkylthio, oxy-C₁₋₃ alkoxy, carboxy, carbamoyl, C₁₋₆ alkyl-carbamoyl, 5- to 7-memberd nitrogen containing heterocyclic group-carbonyl or halogen, (ii) C₁₋₆ acyl, (iii) C₇₋₂₀ aralkyl which may be substituted by halogen, C₁₋₃ alkoxy or C₁₋₄ alkyl, (iv) C₆₋₁₄ aryl which may be substituted by halogen, (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₁₋₆ alkyl-carbonyl, (x) C₃₋₆ cycloalkyloxycarbonyl, or (xi) trifluorosulfonyl, (6) a group of the formula: -S(O)_{f}-R⁶ wherein f is an integer of 0 to 2, and R⁶ is hydrogen or a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by substituent(s) selected from the group consisting of halogen, nitro, cyano, hydroxy, oxo, thioxo, carboxyl, cyano-C₆₋₁₄ aryl, and halogeno-C₆₋₁₄ aryl, (7) a group of the formula: -NR⁹R¹⁰ wherein each of R⁹ and R¹⁰ is hydrogen, C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl, C₁₋₆ acyl or a 5- to 13-membered heterocyclic group containing 1 to 4 hetero atom(s) selected from O, S and N as ring members, (8) a group of the formula: -COR¹¹ wherein R¹¹ is hydrogen, hydroxy, C₁₋₆ alkyl, C₁₋₆ alkoxy, C₃₋₆ cycloalkyl, C₆₋₁₄ aryl, C₇₋₂₀ aralkyl, a group of the formula: -NR⁹R¹⁰ as defined above, or a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (9) a 5- to 13-membered heterocyclic group containing 1 to 4 hetero atom(s) selected from O, S and N as ring members, which may be substituted by halogen, C₁₋₄ alkyl, C₁₋₃ alkoxy, C₁₋₄ alkylthio, or phenoxy which may be substituted by halogen, (10) sulfo, (11) C₆₋₁₄ aryl, (12) C₃₋₇ cycloalkyl, (13) C₁₋₆ alkylenedioxy, (14) oxo, (15) thioxo, (16) C₂₋₄ alkenyl, (17) C₃₋₄ alkynyl, (18) C₃₋₁₀ cycloalkyl, (19) C₂₋₁₀ alkenyl, (20) C₇₋₂₀ aralkyl, (21) amidino and (22) azido.

7. A compound according to Claim 1, wherein R² is a C₁₋₁₀ alkyl, C₃₋₁₀ cycloalkyl, C₇₋₁₀ bicycloalkyl, C₂₋₁₀ alkenyl, C₆₋₁₄ aryl, C₇₋₂₀ aralkyl group, which may be substituted by 1 to 6 substituents selected from the group consisting of (1) halogen, (2) nitro, (3) hydroxy, (4) cyano, (5) C₁₋₄ alkylthio, (6) C₁₋₄ alkoxy, (7) C₁₋₆ alkyl-carbonyloxy or (8) C₃₋₆ cycloalkyl-oxycarbonyl.

8. A compound according to claim 1, wherein R² is hydrogen or a C₁₋₆ alkyl group which may be optionally substituted by C₁₋₆ alkyl-carbonyloxy or C₃₋₆ cycloalkyloxycarbonyl oxy.

9. A compound according to claim 1, wherein R³ is a C₁₋₆ alkyl group which is substituted by a C₁₋₆ alkoxycarbonyl group or a group of the formula: -NH-SO₂-R^{5'}, wherein R^{5'} is a C₁₋₆ alkyl group or a C₆₋₁₄ aryl group.

10. A compound according to claim 1, wherein R³ is a C₁₋₆ alkyl group which is substituted by a group of the formula: -NH-SO₂-R⁵, wherein R⁵ is (1) a C₁₋₆ alkyl group which may optionally be substituted by halogen or (2) a C₆₋₁₄ aryl group.

11. A compound according to claim 1, wherein R³ is a C₁₋₆ alkyl group which is substituted by a group of the formula: -NH-SO₂-R^{5'}, wherein R^{5'} is a C₁₋₆ alkyl group or a C₆₋₁₄ aryl group.

12. A compound according to claim 1, wherein R¹ is a benzyl group which may optionally be substituted by (1) halogen or (2) C₁₋₄ alkylthio,
R² is a hydrogen atom or a C₁₋₄ alkyl group which may optionally be substituted by (1) C₁₋₆ alkyl-carbonyloxy or (2) C₃₋₆ cycloalkyl-oxycarbonyloxy,
R³ is a C₁₋₆ alkyl group which is substituted by (1) a C₁₋₆ alkoxy-carbonyl group or (2) a group of the formula: -NH-SO₂-R^{5"} (wherein R^{5"} is (1) a C₁₋₃ alkyl group which may optionally be substituted by halogen or (2) a phenyl group,
R⁴ is a phenyl group which is substituted by (1) C₁₋₄ alkoxy which may be substituted by C₁₋₆ alkoxy, carboxyl, C₁₋₆ alkyl-carbamoyl, piperazinecarbonyl or halogen, (2) C₇₋₈ aralkyloxy, (3) C₁₋₄ alkyl which may be substituted by C₁₋₃ alkoxy, (4) C₁₋₆ alkanoyl, (5) C₂₋₄ alkenyloxy, (6) C₁₋₆ alkoxy-carbonyl or (7) C₁₋₆ carbamoyl.

13. 2,4(1H,3H)-dioxo-6-(4-methoxymethoxyphenyl)-1-(2-methylthiobenzyl)-5-(methanesulfonamidomethyl)thieno[2,3-d]pyrimidine-3-acetic acid or its salt.

14. 2,4(1H,3H)-dioxo-6-(4-methoxymethoxyphenyl)-1-(2-methylthiobenzyl)-5-(ethanesulfonamidomethyl)thieno[2,3-d]pyrimidine-3-acetic acid or its salt.

15. 2,4(1H,3H)-dioxo-6-(4-methoxyphenyl)-1-(2-methylthiobenzyl)-5-(methanesulfonamidomethyl)thieno[2,3-d]pyrimidine-3-acetic acid or its salt.

16. Ethyl 2,4(1H,3H)-dioxo-6-(4-methoxyphenyl)-1-(2-methylthiobenzyl)-5-(carboxymethyl)thieno[2,3-d]pyrimidine-3-acetate.

17. A method for producing a compound as defined in claim 1, which comprises subjecting a compound of the formula: 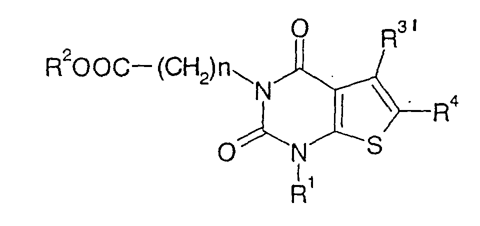 wherein R^{3'} is a C₁₋₆ alkyl group which is halogenated or cyanated, and other symbols have the same meaning as defined in claim 1, to (1) a nucleophilic substitution reaction with a sulfonamide compound when R^{3'} is a C₁₋₆ alkyl group which is halogenated or (2) alkali-hydrolysis and the esterification when R^{3'} is a C₁₋₆ alkyl group which is cyanated.

18. A pharmaceutical composition, which comprises a compound as defined in claim 1 or 16 and a carrier, excipient or diluent therefor.

19. A pharmaceutical composition according to claim 18, which is a therapeutic drug for treating vasoconstriction in a mammal.

20. A pharmaceutical composition according to claim 19, wherein the vasoconstriction is in a coronary artery, coronary vein, cerebrovascular system or pulmonary vascular system.

21. A pharmaceutical composition according to claim 18, which is for antagonizing endothelin activity.

22. A pharmaceutical composition according to claim 21, which is a therapeutic drug for acute renal insufficiency, cardiac infarction or liver insufficiency.

23. A pharmaceutical composition according to claim 21, which is a treapeutic drug for hypofunction of an organ caused by a surgery or transplant.

24. A pharmaceutical composition according to claim 23, wherein the organ is liver.

25. Use of a compound as defined in claim 1 or 16 for producing a pharmaceutical composition for the manufacture of a medicament for therapeutic application on vasoconstriction.

26. Use of a compound as defined in claim 1 or 16 for producing a pharmaceutical composition for the manufacture of a medicament for therapeutic application on acute renal insufficiency, cardiac infarction or liver insufficiency.

## Patentansprüche

1. Verbindung der Formel 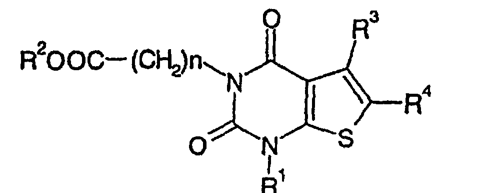 worin R¹ und R² jeweils (a) Wasserstoff oder (b) eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe sind, die durch 1 bis 6 Substituenten substituiert sein können, die aus der Gruppe ausgewählt sind, die aus (1) Halogen, (2) Nitro, (3) Nitroso, (4) Cyan, (5) Hydroxy, das durch (i) C₁₋₆-Alkyl, das durch Hydroxy, C₁₋₆-Alkoxy, C₁₋₃-Alkoxy-C₁₋₃-alkoxy, C₁₋₃-Alkylthio, Oxy-C₁₋₃-alkoxy, Carboxy, Carbamoyl, C₁₋₆-Alkylcarbamoyl, (5- bis 7gliedrige, stickstoffhaltige heterocyclische Gruppe)-Carbonyl oder Halogen substituiert sein kann, (ii) C₁₋₆-Acyl, (iii) C₇₋₂₀-Aralkyl, das durch Halogen, C₁₋₃-Alkoxy oder C₁₋₄-Alkyl substituiert sein kann, (iv) C₆₋₁₄-Aryl, das durch Halogen substituiert sein kann, (v) C₂₋₆-Alkenyl, (vi) C₃₋₇-Cycloalkyl, (vii) C₁₋₃-Alkoxycarbonyl, (viii) Mono- oder Di-C₁₋₆-alkylamino, (ix) C₁₋₆-Alkylcarbonyl, (x) C₃₋₆-Cycloalkyloxycarbonyl oder (xi) Trifluorsulfonyl substituiert sein kann, (6) einer Gruppe der Formel -S(O)_{f}-R⁶, worin f eine ganze Zahl von 0 bis 2 ist und R⁶ Wasserstoff oder eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch (einen) Substituenten substituiert sein können, die aus der aus Halogen, Nitro, Cyan, Hydroxy, Oxo, Thioxo, Carboxy, Cyan-C₆₋₁₄-aryl und Halogen-C₆₋₁₄-aryl bestehenden Gruppe ausgewählt sind, (7) einer Gruppe der Formel -NR⁹R¹⁰, worin R⁹ und R¹⁰ jeweils Wasserstoff, eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl-, C₇₋₂₀-Aralkyl-, C₁₋₆-Acyl- oder eine 5- bis 13gliedrige heterocyclische Gruppe sind, die 1 bis 4 aus O, S und N ausgewählte(s) Heteroatom(e) als Ringglieder enthält, (8) einer Gruppe der Formel -COR¹¹, worin R¹¹ Wasserstoff, Hydroxy, eine C₁₋₆-Alkyl-, C₁₋₆-Alkoxy-, C₃₋₆-Cycloalkyl-, C₆₋₁₄-Aryl-, C₇₋₂₀-Aralkyl-, eine wie vorstehend definierte Gruppe der Formel -NR⁹R¹⁰ oder eine 5- bis 13gliedrige heterocyclische Gruppe ist, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (9) einer 5- bis 13gliedrigen heterocyclischen Gruppe, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (10) Sulfo, (11) C₆₋₁₄-Aryl, (12) C₃₋₇-Cycloalkyl, (13) C₁₋₆-Alkylendioxy, (14) Oxo, (15) Thioxo, (16) C₂₋₄-Alkenyl, (17) C₃₋₄-Alkinyl, (18) C₃₋₁₀-Cycloalkyl, (19) C₂₋₁₀-Alkenyl, (20) C₇₋₂₀-Aralkyl, (21) Amidino und (22) Azido besteht;
R³ eine C₁₋₆-Alkylgruppe ist, die durch C₁₋₆-Alkoxycarbonyl oder eine Gruppe der Formel -NH-SO₂-R⁵ substituiert ist, worin R⁵ (1) C₁₋₆-Alkyl, das durch Halogen substituiert sein kann oder (2) C₆₋₁₄-Aryl ist;
R⁴ eine Phenylgruppe ist, die durch (1) C₁₋₄-Alkoxy, das durch C₁₋₆-Alkoxy, Carboxy, C₁₋₆-Alkylcarbamoyl, Piperazincarbonyl oder Halogen substituiert sein kann, (2) C₇₋₈-Aralkyloxy, (3) C₁₋₄-Alkyl, das durch Hydroxy, Oxo oder C₁₋₃-Alkoxy substituiert sein kann, (4) C₁₋₆-Alkanoyl, (5) C₂₋₄-Alkenyloxy, (6) C₁₋₆-Alkoxycarbonyl oder (7) C₁₋₆-Alkylcarbamoyl substituiert sein kann, und
n eine ganze Zahl von 1 bis 3 ist,
oder ein Salz davon.

2. Verbindung gemäß Anspruch 1, wobei R¹ eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch 1 bis 6 Substituenten substituiert sein können, die aus der Gruppe ausgewählt sind, die aus (1) Halogen, (2) Nitro, (3) Nitroso, (4) Cyan, (5) Hydroxy, das durch (i) C₁₋₆-Alkyl, das durch Hydroxy, C₁₋₆-Alkoxy, C₁₋₃-Alkoxy-C₁₋₃-alkoxy, C₁₋₃-Alkylthio, Oxy-C₁₋₃-alkoxy, Carboxy, Carbamoyl, C₁₋₆-Alkylcarbamoyl, (5- bis 7gliedrige, stickstoffhaltige heterocyclische Gruppe)-Carbonyl oder Halogen substituiert sein kann, (ii) C₁₋₆-Acyl, (iii) C₇₋₂₀-Aralkyl, das durch Halogen, C₁₋₃-Alkoxy oder C₁₋₄-Alkyl substituiert sein kann, (iv) C₆₋₁₄-Aryl, das durch Halogen substituiert sein kann, (v) C₂₋₆-Alkenyl, (vi) C₃₋₇-Cycloalkyl, (vii) C₁₋₃-Alkoxycarbonyl, (viii) Mono- oder Di-C₁₋₆-alkylamino, (ix) C₁₋₆-Alkylcarbonyl, (x) C₃₋₆-Cycloalkyloxycarbonyl oder (xi) Trifluorsulfonyl substituiert sein kann, (6) einer Gruppe der Formel -S(O)_{f}-R⁶, worin f eine ganze Zahl von 0 bis 2 ist und R⁶ Wasserstoff oder eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch (einen) Substituenten substituiert sein können, die aus der aus Halogen, Nitro, Cyan, Hydroxy, Oxo, Thioxo, Carboxy, Cyan-C₆₋₁₄-aryl und Halogen-C₆₋₁₄-aryl bestehenden Gruppe ausgewählt sind, (7) einer Gruppe der Formel -NR⁹R¹⁰, worin R⁹ und R¹⁰ jeweils Wasserstoff, eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl-, C₇₋₂₀-Aralkyl-, C₁₋₆-Acyl- oder eine 5- bis 13gliedrige heterocyclische Gruppe sind, die 1 bis 4 aus O, S und N ausgewählte(s) Heteroatom(e) als Ringglieder enthält, (8) einer Gruppe der Formel -COR¹¹, worin R¹¹ Wasserstoff, Hydroxy, eine C₁₋₆-Alkyl-, C₁₋₆-Alkoxy-, C₃₋₆-Cycloalkyl-, C₆₋₁₄-Aryl-, C₇₋₂₀-Aralkyl-, eine wie vorstehend definierte Gruppe der Formel -NR⁹R¹⁰ oder eine 5- bis 13gliedrige heterocyclische Gruppe ist, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (9) einer 5- bis 13gliedrigen heterocyclischen Gruppe, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (10) Sulfo, (11) C₆₋₁₄-Aryl, (12) C₃₋₇-Cycloalkyl, (13) C₁₋₆-Alkylendioxy, (14) Oxo, (15) Thioxo, (16) C₂₋₄-Alkenyl, (17) C₃₋₄-Alkinyl, (18) C₃₋₁₀-Cycloalkyl, (19) C₂₋₁₀-Alkenyl, (20) C₇₋₂₀-Aralkyl, (21) Amidino und (22) Azido besteht.

3. Verbindung gemäß Anspruch 1, wobei R¹ eine C₇₋₂₀-Aralkylgruppe ist, die durch Halogen, Nitro, Hydroxy, Cyan, C₁₋₄-Alkyl, C₁₋₄-Alkylthio oder C₁₋₄-Alkoxy substituiert sein kann.

4. Verbindung gemäß Anspruch 1, wobei R¹ Benzyl ist, das durch Halogen oder C₁₋₄-Alkylthio substituiert sein kann.

5. Verbindung gemäß Anspruch 1, wobei R² eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch 1 bis 6 Substituenten substituiert sein können, die aus der Gruppe ausgewählt sind, die aus (1) Halogen, (2) Nitro, (3) Nitroso, (4) Cyan, (5) Hydroxy, das durch (i) C₁₋₆-Alkyl, das durch Hydroxy, C₁₋₆-Alkoxy, C₁₋₃-Alkoxy-C₁₋₃-alkoxy, C₁₋₃-Alkylthio, Oxy-C₁₋₃-alkoxy, Carboxy, Carbamoyl, C₁₋₆-Alkylcarbamoyl, (5- bis 7gliedrige, stickstoffhaltige heterocyclische Gruppe)-Carbonyl oder Halogen substituiert sein kann, (ii) C₁₋₆-Acyl, (iii) C₇₋₂₀-Aralkyl, das durch Halogen, C₁₋₃-Alkoxy oder C₁₋₄-Alkyl substituiert sein kann, (iv) C₆₋₁₄-Aryl, das durch Halogen substituiert sein kann, (v) C₂₋₆-Alkenyl, (vi) C₃₋₇-Cycloalkyl, (vii) C₁₋₃-Alkoxycarbonyl, (viii) Mono- oder Di-C₁₋₆-alkylamino, (ix) C₁₋₆-Alkylcarbonyl, (x) C₃₋₆-Cycloalkyloxycarbonyl oder (xi) Trifluorsulfonyl substituiert sein kann, (6) einer Gruppe der Formel -S(O)_{f}-R⁶, worin f eine ganze Zahl von 0 bis 2 ist und R⁶ Wasserstoff oder eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch (einen) Substituenten substituiert sein können, die aus der aus Halogen, Nitro, Cyan, Hydroxy, Oxo, Thioxo, Carboxy, Cyan-C₆₋₁₄-aryl und Halogen-C₆₋₁₄-aryl bestehenden Gruppe ausgewählt sind, (7) einer Gruppe der Formel -NR⁹R¹⁰, worin R⁹ und R¹⁰ jeweils Wasserstoff, eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl-, C₇₋₂₀-Aralkyl-, C₁₋₆-Acyl- oder eine 5- bis 13gliedrige heterocyclische Gruppe sind, die 1 bis 4 aus O, S und N ausgewählte(s) Heteroatom(e) als Ringglieder enthält, (8) einer Gruppe der Formel -COR¹¹, worin R¹¹ Wasserstoff, Hydroxy, eine C₁₋₆-Alkyl-, C₁₋₆-Alkoxy-, C₃₋₆-Cycloalkyl-, C₆₋₁₄-Aryl-, C₇₋₂₀-Aralkyl-, eine wie vorstehend definierte Gruppe der Formel -NR⁹R¹⁰ oder eine 5- bis 13gliedrige heterocyclische Gruppe ist, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (9) einer 5- bis 13gliedrigen heterocyclischen Gruppe, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (10) Sulfo, (11) C₆₋₁₄-Aryl, (12) C₃₋₇-Cycloalkyl, (13) C₁₋₆-Alkylendioxy, (14) Oxo, (15) Thioxo, (16) C₂₋₄-Alkenyl, (17) C₃₋₄-Alkinyl, (18) C₃₋₁₀-Cycloalkyl, (19) C₂₋₁₀-Alkenyl, (20) C₇₋₂₀-Aralkyl, (21) Amidino und (22) Azido besteht.

6. Verbindung gemäß Anspruch 1, wobei R² eine C₁₋₁₀-Alkylgruppe ist, die durch 1 bis 6 Substituenten substituiert sein kann, die aus der Gruppe ausgewählt sind, die aus (1) Halogen, (2) Nitro, (3) Nitroso, (4) Cyan, (5) Hydroxy, das durch (i) C₁₋₆-Alkyl, das durch Hydroxy, C₁₋₆-Alkoxy, C₁₋₃-Alkoxy-C₁₋₃-alkoxy, C₁₋₃-Alkylthio, Oxy-C₁₋₃-alkoxy, Carboxy, Carbamoyl, C₁₋₆-Alkylcarbamoyl, (5- bis 7gliedrige, stickstoffhaltige heterocyclische Gruppe)-Carbonyl oder Halogen substituiert sein kann, (ii) C₁₋₆-Acyl, (iii) C₇₋₂₀-Aralkyl, das durch Halogen, C₁₋₃-Alkoxy oder C₁₋₄-Alkyl substituiert sein kann, (iv) C₆₋₁₄-Aryl, das durch Halogen substituiert sein kann, (v) C₂₋₆-Alkenyl, (vi) C₃₋₇-Cycloalkyl, (vii) C₁₋₃-Alkoxycarbonyl, (viii) Mono- oder Di-C₁₋₆-alkylamino, (ix) C₁₋₆-Alkylcarbonyl, (x) C₃₋₆-Cycloalkyloxycarbonyl oder (xi) Trifluorsulfonyl substituiert sein kann, (6) einer Gruppe der Formel -S(O)_{f}-R⁶, worin f eine ganze Zahl von 0 bis 2 ist und R⁶ Wasserstoff oder eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch (einen) Substituenten substituiert sein können, die aus der aus Halogen, Nitro, Cyan, Hydroxy, Oxo, Thioxo, Carboxy, Cyan-C₆₋₁₄-aryl und Halogen-C₆₋₁₄-aryl bestehenden Gruppe ausgewählt sind, (7) einer Gruppe der Formel -NR⁹R¹⁰, worin R⁹ und R¹⁰ jeweils Wasserstoff, eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl-, C₇₋₂₀-Aralkyl-, C₁₋₆-Acyl- oder eine 5- bis 13gliedrige heterocyclische Gruppe sind, die 1 bis 4 aus O, S und N ausgewählte(s) Heteroatom(e) als Ringglieder enthält, (8) einer Gruppe der Formel -COR¹¹, worin R¹¹ Wasserstoff, Hydroxy, eine C₁₋₆-Alkyl-, C₁₋₆-Alkoxy-, C₃₋₆-Cycloalkyl-, C₆₋₁₄-Aryl-, C₇₋₂₀-Aralkyl-, eine wie vorstehend definierte Gruppe der Formel -NR⁹R¹⁰ oder eine 5- bis 13gliedrige heterocyclische Gruppe ist, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (9) einer 5- bis 13gliedrigen heterocyclischen Gruppe, die 1 bis 4 aus O, S und N ausgewählte(s) Heteroatom(e) als Ringglieder enthält und durch Halogen, C₁₋₄-Alkyl, C₁₋₃-Alkoxy, C₁₋₄-Alkylthio oder Phenoxy, das durch Halogen substituiert sein kann, substituiert sein kann, (10) Sulfo, (11) C₆₋₁₄-Aryl, (12) C₃₋₇-Cycloalkyl, (13) C₁₋₆-Alkylendioxy, (14) Oxo, (15) Thioxo, (16) C₂₋₄-Alkenyl, (17) C₃₋₄-Alkinyl, (18) C₃₋₁₀-Cycloalkyl, (19) C₂₋₁₀-Alkenyl, (20) C₇₋₂₀-Aralkyl, (21) Amidino und (22) Azido besteht.

7. Verbindung gemäß Anspruch 1, wobei R² eine C₁₋₁₀-Alkyl-, C₃₋₁₀-Cycloalkyl-, C₇₋₁₀-Bicycloalkyl-, C₂₋₁₀-Alkenyl-, C₆₋₁₄-Aryl- oder C₇₋₂₀-Aralkylgruppe ist, die durch 1 bis 6 Substituenten substituiert sein können, die aus der Gruppe ausgewählt sind, die aus (1) Halogen, (2) Nitro, (3) Hydroxy, (4) Cyan, (5) C₁₋₄-Alkylthio, (6) C₁₋₄-Alkoxy, (7) C₁₋₆-Alkylcarbonyloxy oder (8) C₃₋₆-Cycloalkyloxycarbonyl besteht.

8. Verbindung gemäß Anspruch 1, wobei R² Wasserstoff oder eine C₁₋₆-Alkylgruppe ist, die gegebenenfalls durch C₁₋₆-Alkylcarbonyloxy oder C₃₋₆-Cycloalkyloxycarbonyloxy substituiert sein kann.

9. Verbindung gemäß Anspruch 1, wobei R³ eine C₁₋₆-Alkylgruppe ist, die durch eine C₁₋₆-Alkoxycarbonylgruppe oder eine Gruppe der Formel -NH-SO₂-R^{5'} ist, worin R^{5'} eine C₁₋₆-Alkylgruppe oder eine C₆₋₁₄-Arylgruppe ist, substituiert ist.

10. Verbindung gemäß Anspruch 1, wobei R³ eine C₁₋₆-Alkylgruppe ist, die durch eine Gruppe der Formel -NH-SO₂-R⁵, worin R⁵ (1) eine C₁₋₆-Alkylgruppe, die gegebenenfalls durch Halogen substituiert sein kann, oder (2) eine C₆₋₁₄-Arylgruppe ist, substituiert ist.

11. Verbindung gemäß Anspruch 1, wobei R³ eine C₁₋₆-Alkylgruppe ist, die durch eine Gruppe der Formel -NH-SO₂-R^{5'}, worin R^{5'} eine C₁₋₆-Alkylgruppe oder eine C₆₋₁₄-Arylgruppe ist, substituiert ist.

12. Verbindung gemäß Anspruch 1, wobei R¹ eine Benzylgruppe ist, die gegebenenfalls durch (1) Halogen oder (2) C₁₋₄-Alkylthio substituiert sein kann,
R² ein Wasserstoffatom oder eine C₁₋₄-Alkylgruppe ist, die gegebenenfalls durch (1) C₁₋₆-Alkylcarbonyloxy oder (2) C₃₋₆-Cycloalkyloxycarbonyloxy substituiert sein kann,
R³ eine C₁₋₆-Alkylgruppe ist, die durch (1) eine C₁₋₆-Alkoxycarbonylgruppe oder (2) eine Gruppe der Formel -NH-SO₂-R^{5"} (worin R^{5"} (1) eine C₁₋₃-Alkylgruppe, die gegebenenfalls durch Halogen substituiert sein kann, oder (2) eine Phenylgruppe ist) substituiert ist,
R⁴ eine Phenylgruppe ist, die durch (1) C₁₋₄-Alkoxy, das durch C₁₋₆-Alkoxy, Carboxy, C₁₋₆-Alkylcarbamoyl, Piperazincarbonyl oder Halogen substituiert sein kann, (2) C₇₋₈-Aralkyloxy, (3) C₁₋₄-Alkyl, das durch C₁₋₃-Alkoxy substituiert sein kann, (4) C₁₋₆-Alkanoyl, (5) C₂₋₄-Alkenyloxy, (6) C₁₋₆-Alkoxycarbonyl oder (7) C₁₋₆-Carbamoyl substituiert sein kann.

13. 2,4(1H,3H)-Dioxo-6-(4-methoxymethoxyphenyl)-1-(2-methylthiobenzyl)-5-(methansulfonamidomethyl)-thieno[2,3-d]pyrimidin-3-essigsäure oder ihr Salz.

14. 2,4(1H,3H)-Dioxo-6-(4-methoxymethoxyphenyl)-1-(2-methylthiobenzyl)-5-(ethansulfonamidomethyl)-thieno[2,3-d]pyrimidin-3-essigsäure oder ihr Salz.

15. 2,4(1H,3H)-Dioxo-6-(4-methoxyphenyl)-1-(2-methylthiobenzyl)-5-(methansulfonamidomethyl)-thieno[2,3-d]pyrimidin-3-essigsäure oder ihr Salz.

16. Ethyl-2,4(1H,3H)-dioxo-6-(4-methoxyphenyl)-1-(2-methylthiobenzyl)-5-(carboxymethyl)thieno[2,3-d]pyrimidin-3-acetat.

17. Verfahren zum Herstellen einer in Anspruch 1 definierten Verbindung, die das Unterziehen einer Verbindung der Formel 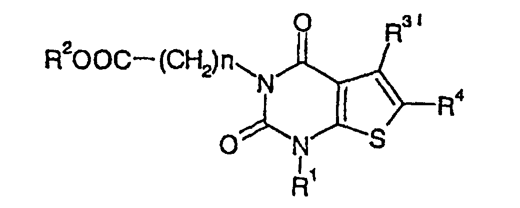 worin R^{3'} eine C₁₋₆-Alkylgruppe ist, die halogeniert oder cyaniert ist, und die anderen Symbole dieselbe Bedeutung wie in Anspruch 1 aufweisen, (1) einer nukleophilen Substitutionsreaktion mit einer Sulfonamidverbindung, wenn R^{3'} eine C₁₋₆-Alkylgruppe ist, die halogeniert ist, oder (2) Alkalihydrolyse und der Veresterung umfaßt, wenn R^{3'} eine C₁₋₆-Alkylgruppe ist, die cyaniert ist.

18. Pharmazeutische Zusammensetzung, die eine in Anspruch 1 oder 16 definierte Verbindung und einen Träger, Arzneimittelträger oder Verdünnungsmittel dafür umfaßt.

19. Pharmazeutische Zusammensetzung gemäß Anspruch 18, die ein therapeutischer Wirkstoff zum Behandeln einer Gefäßverengung in einem Säuger ist.

20. Pharmazeutische Zusammensetzung gemäß Anspruch 19, wobei die Gefäßverengung in einer Koronararterie, Koronarvene, dem zerebrovaskulären System oder Lungengefäßsystem besteht.

21. Pharmazeutische Zusammensetzung gemäß Anspruch 18 zum Antagonisieren einer Endothelinaktivität.

22. Pharmazeutische Zusammensetzung gemäß Anspruch 21, die ein therapeutischer Wirkstoff bei akuter Niereninsuffizienz, Herzinfarkt oder Leberinsuffizienz ist.

23. Pharmazeutische Zusammensetzung.gemäß Anspruch 21, die ein therapeutischer Wirkstoff bei durch eine Operation oder Transplantation verursachter Organunterfunktion ist.

24. Pharmazeutische Zusammensetzung gemäß Anspruch 23, wobei das Organ die Leber ist.

25. Verwendung einer in Anspruch 1 oder 16 definierten Verbindung zum Herstellen einer pharmazeutischen Zusammensetzung zur Herstellung eines Arzneimittels zur therapeutischen Anwendung gegen Gefäßverengung.

26. Verwendung einer in Anspruch 1 oder 16 definierten Verbindung zum Herstellen einer pharmazeutischen Zusammensetzung zur Herstellung eines Arzneimittels zur therapeutischen Anwendung gegen akute Niereninsuffizienz, Herzinfarkt oder Leberinsuffizienz.

## Revendications

1. Composé de formule 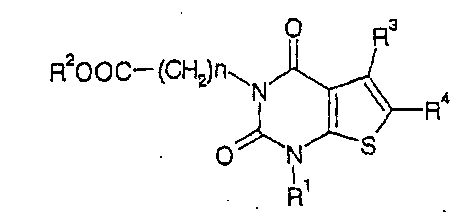 dans laquelle R¹ et R² représentent chacun (a) un atome d'hydrogène ou (b) un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement de 1 à 6 substituants choisis dans le groupe formé par les radicaux
(1) halogéno,
(2) nitro,
(3) nitroso,
(4) cyano,
(5) hydroxy portant éventuellement des substituants
(i) alkyle en C₁₋₆ substitué éventuellement par des groupes hydroxy, alcoxy en C₁₋₆, (alcoxy en C₁₋₃)-(alcoxy en C₁₋₃), alkylthio en C₁₋₃, oxy(alcoxy en C₁₋₃), carboxy, carbamoyle, (alkyle en C₁₋₆)-carbamoyle, hétérocycle azoté à 5 - 7 chaînons-carbonyle ou halogéno,
(ii) acyle en C₁₋₆,
(iii) aralkyle en C₇₋₂₀ portant éventuellement des substituants halogéno, alcoxy en C₁₋₃ ou alkyle en C₁₋₄,
(iv) aryle en C₆₋₁₄ éventuellement halogéné,
(v) alcényle en C₂₋₆,
(vi) cycloalkyle en C₃₋₇,
(vii) (alcoxy en C₁₋₃)-carbonyle,
(viii) mono- ou di-(alkyle en C₁₋₆)-amino,
(ix) (alkyle en C₁₋₆)-carbonyle,
(x) (cycloalkyle en C₃₋₆)oxycarbonyle, ou
(xi) trifluorosulfonyle,
(6) un groupe de formule -S(O)_{f}-R⁶ où f représente un nombre entier compris entre 0 et 2, et R⁶ représente un atome d'hydrogène ou un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement un ou plusieurs substituants choisis dans le groupe formé par halogéno, nitro, cyano, hydroxy, oxo, thioxo, carboxyle, cyano-(aryle en C₆₋₁₄) et halogéno-(aryle en C₆₋₁₄),
(7) un groupe de formule -NR⁹R¹⁰ où R⁹ et R¹⁰ représentent chacun un atome d'hydrogène, un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, acyle en C₁₋₆ ou hétérocyclique à 5 -13 chaînons contenant dans leur cycle de 1 à 4 hétéroatomes choisis parmi O, S et N,
(8) un groupe de formule : -COR¹¹ où R¹¹ représente un atome d'hydrogène, un groupe hydroxy, alkyle en C₁₋₆, alcoxy en C₁₋₆, cycloalkyle en C₃₋₆, aryle en C₆₋₁₄, aralkyle en C₇₋₂₀, un groupe de formule -NR⁹R¹⁰ tel que défini ci-dessus, ou un groupe hétérocyclique à 5 -13 chaînons contenant dans son cycle de 1 à 4 hétéroatomes choisis parmi O, S et N, éventuellement substitué par des groupes halogéno, alkyle en C₁₋₄, alcoxy en C₁₋₃, alkylthio en C₁₋₄ ou phénoxy éventuellement halogéné,
(9) un groupe hétérocyclique à 5 -13 chaînons contenant dans son cycle de 1 à 4 hétéroatomes choisis parmi O, S et N, qui peut porter un ou plusieurs substituants halogéno, alkyle en C₁₋₄, alcoxy en C₁₋₃, alkylthio en C₁₋₄, ou phénoxy éventuellement halogéné,
(10) sulfo,
(11) aryle en C₆₋₁₄,
(12) cycloalkyle en C₃₋₇,
(13) alkylènedioxy en C₁₋₆,
(14) oxo,
(15) thioxo,
(16) alcényle en C₂₋₄,
(17) alcynyle en C₃₋₄,
(18) cycloalkyle en C₃₋₁₀,
(19) alcényle en C₂₋₁₀,
(20) aralkyle en C₇₋₂₀,
(21) amidino et
(22) azido ;
R³ représente un groupe alkyle en C₁₋₆ portant un substituant alcoxy en C₁₋₆ ou un groupe de formule -NH-SO₂-R⁵ où R⁵ représente (1) un groupe alkyle en C₁₋₆ éventuellement halogéné ou (2) un groupe aryle en C₆₋₁₄ ;
R⁴ représente un groupe phényle portant éventuellement un ou plusieurs substituants (1) alcoxy en C₁₋₄, à son tour éventuellement substitué par des groupes alcoxy en C₁₋₆, carboxy, (alkyle en C₁₋₆)-carbamoyle, pipérazinecarbonyle ou halogéno, (2) aralkyloxy en C₇₋₈, (3) alkyle en C₁₋₄ portant éventuellement un substituant hydroxy, oxo ou alcoxy en C₁₋₃, (4) alcanoyle en C₁₋₆, (5) alcényloxy en C₂₋₄, (6) (alcoxy en C₁₋₆)-carbonyle ou (7) (alkyle en C₁₋₆)-carbamoyle, et
n est un nombre entier compris entre 1 et 3,
ou un sel d'un tel composé.

2. Composé selon la revendication 1, dans lequel R¹ représente un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement de 1 à 6 substituants choisis dans le groupe formé par les radicaux
(1) halogéno,
(2) nitro,
(3) nitroso,
(4) cyano,
(5) hydroxy portant éventuellement des substituants
(i) alkyle en C₁₋₆ substitué éventuellement par des groupes hydroxy, alcoxy en C₁₋₆, (alcoxy en C₁₋₃)-(alcoxy en C₁₋₃), alkylthio en C₁₋₃, oxy(alcoxy en C₁₋₃), carboxy, carbamoyle, (alkyle en C₁₋₆)-carbamoyle, hétérocycle azoté à 5 - 7 chaînons-carbonyle ou halogéno,
(ii) acyle en C₁₋₆,
(iii) aralkyle en C₇₋₂₀ portant éventuellement des substituants halogéno, alcoxy en C₁₋₃ ou alkyle en C₁₋₄,
(iv) aryle en C₆₋₁₄ éventuellement halogéné,
(v) alcényle en C₂₋₆,
(vi) cycloalkyle en C₃₋₇,
(vii) (alcoxy en C₁₋₃)-carbonyle,
(viii) mono- ou di-(alkyle en C₁₋₆)-amino,
(ix) (alkyle en C₁₋₆)-carbonyle,
(x) (cycloalkyle en C₃₋₆)oxycarbonyle, ou
(xi) trifluorosulfonyle,
(6) un groupe de formule -S(O)_{f}-R⁶ où f représente un nombre entier compris entre 0 et 2, et R⁶ représente un atome d'hydrogène ou un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement un ou plusieurs substituants choisis dans le groupe formé par halogéno, nitro, cyano, hydroxy, oxo, thioxo, carboxyle, cyano-(aryle en C₆₋₁₄) et halogéno-(aryle en C₆₋₁₄),
(7) un groupe de formule -NR⁹R¹⁰ où R⁹ et R¹⁰ représentent chacun un atome d'hydrogène, un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, acyle en C₁₋₆ ou hétérocyclique à 5 -13 chaînons contenant dans son cycle de 1 à 4 hétéroatomes choisis parmi O, S et N,
(8) un groupe de formule : -COR¹¹ où R¹¹ représente un atome d'hydrogène, un groupe hydroxy, alkyle en C₁₋₆, alcoxy en C₁₋₆, cycloalkyle en C₃₋₆, aryle en C₆₋₁₄, aralkyle en C₇₋₂₀, un groupe de formule -NR⁹R¹⁰ tel que défini ci-dessus, ou un groupe hétérocyclique à 5 -13 chaînons contenant dans son cycle de 1 à 4 hétéroatomes choisis parmi O, S et N, éventuellement substitué par des groupes halogéno, alkyle en C₁₋₄, alcoxy en C₁₋₃, alkylthio en C₁₋₄ ou phénoxy éventuellement halogéné,
(9) un groupe hétérocyclique à 5 -13 chaînons contenant dans son cycle de 1 à 4 hétéroatomes choisis parmi O, S et N, qui peut porter un ou plusieurs substituants halogéno, alkyle en C₁₋₄, alcoxy en C₁₋₃, alkylthio en C₁₋₄, ou phénoxy éventuellement halogéné,
(10) sulfo,
(11) aryle en C₆₋₁₄,
(12) cycloalkyle en C₃₋₇,
(13) alkylènedioxy en C₁₋₆,
(14) oxo,
(15) thioxo,
(16) alcényle en C₂₋₄,
(17) alcynyle en C₃₋₄,
(18) cycloalkyle en C₃₋₁₀,
(19) alcényle en C₂₋₁₀,
(20) aralkyle en C₇₋₂₀,
(21) amidino et
(22) azido.

3. Composé selon la revendication 1, dans lequel R¹ représente un groupe aralkyle en C₇₋₂₀ portant éventuellement un ou plusieurs substituants halogéno, nitro, hydroxy, cyano, alkyle en C₁₋₄, alkylthio en C₁₋₄ ou alcoxy en C₁₋₄.

4. Composé selon la revendication 1, dans lequel R¹ représente un groupe benzyle portant éventuellement un ou plusieurs substituants halogéno ou alkylthio en C₁₋₄.

5. Composé selon la revendication 1, dans lequel R² représente un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement de 1 à 6 substituants choisis dans le groupe formé par les radicaux
(1) halogéno,
(2) nitro,
(3) nitroso,
(4) cyano,
(5) hydroxy portant éventuellement des substituants
(i) alkyle en C₁₋₆ substitué éventuellement par des groupes hydroxy, alcoxy en C₁₋₆, (alcoxy en C₁₋₃)-(alcoxy en C₁₋₃), alkylthio en C₁₋₃, oxy(alcoxy en C₁₋₃), carboxy, carbamoyle, (alkyle en C₁₋₆)-carbamoyle, hétérocycle azoté à 5 - 7 chaînons-carbonyle ou halogéno,
(ii) acyle en C₁₋₆,
(iii) aralkyle en C₇₋₂₀ portant éventuellement des substituants halogéno, alcoxy en C₁₋₃ ou alkyle en C₁₋₄,
(iv) aryle en C₆₋₁₄ éventuellement halogéné,
(v) alcényle en C₂₋₆,
(vi) cycloalkyle en C₃₋₇,
(vii) (alcoxy en C₁₋₃)-carbonyle,
(viii) mono- ou di-(alkyle en C₁₋₆)-amino,
(ix) (alkyle en C₁₋₆)-carbonyle,
(x) (cycloalkyle en C₃₋₆)oxycarbonyle, ou
(xi) trifluorosulfonyle,
(6) un groupe de formule -S(O)_{f}R⁶ où f représente un nombre entier compris entre 0 et 2, et R⁶ représente un atome d'hydrogène ou un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement un ou plusieurs substituants choisis dans le groupe formé par halogéno, nitro, cyano, hydroxy, oxo, thioxo, carboxyle, cyano-(aryle en C₆₋₁₄) et halogéno-(aryle en C₆₋₁₄),
(7) un groupe de formule -NR⁹R¹⁰ où R⁹ et R¹⁰ représentent chacun un atome d'hydrogène, un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, acyle en C₁₋₆ ou hétérocyclique à 5 -13 chaînons contenant dans son cycle de 1 à 4 hétéroatomes choisis parmi O, S et N,
(8) un groupe de formule : -COR¹¹ où R¹¹ représente un atome d'hydrogène, un groupe hydroxy, alkyle en C₁₋₆, alcoxy en C₁₋₆, cycloalkyle en C₃₋₆, aryle en C₆₋₁₄, aralkyle en C₇₋₂₀, un groupe de formule -NR⁹R¹⁰ tel que défini ci-dessus, ou un groupe hétérocyclique à 5 -13 chaînons contenant dans son cycle de 1 à 4 hétéroatomes choisis parmi O, S et N, éventuellement substitué par des groupes halogéno, alkyle en C₁₋₄, alcoxy en C₁₋₃, alkylthio en C₁₋₄ ou phénoxy éventuellement halogéné,
(9) un groupe hétérocyclique à 5 -13 chaînons contenant dans son cycle de 1 à 4 hétéroatomes choisis parmi O, S et N, qui peut porter un ou plusieurs substituants halogéno, alkyle en C₁₋₄, alcoxy en C₁₋₃, alkylthio en C₁₋₄, ou phénoxy éventuellement halogéné,
(10) sulfo,
(11) aryle en C₆₋₁₄,
(12) cycloalkyle en C₃₋₇,
(13) alkylènedioxy en C₁₋₆,
(14) oxo,
(15) thioxo,
(16) alcényle en C₂₋₄,
(17) alcynyle en C₃₋₄,
(18) cycloalkyle en C₃₋₁₀,
(19) alcényle en C₂₋₁₀,
(20) aralkyle en C₇₋₂₀,
(21) amidino et
(22) azido.

6. Composé selon la revendication 1, dans lequel R² représente un groupe alkyle en C₁₋₁₀ portant éventuellement de 1 à 6 substituants choisis dans le groupe formé par les radicaux
(1) halogéno,
(2) nitro,
(3) nitroso,
(4) cyano,
(5) hydroxy portant éventuellement des substituants
(i) alkyle en C₁₋₆ substitué éventuellement par des groupes hydroxy, alcoxy en C₁₋₆, (alcoxy en C₁₋₃)-(alcoxy en C₁₋₃), alkylthio en C₁₋₃, oxy(alcoxy en C₁₋₃), carboxy, carbamoyle, (alkyle en C₁₋₆)-carbamoyle, hétérocycle azoté à 5 - 7 chaînons-carbonyle ou halogéno,
(ii) acyle en C₁₋₆,
(iii) aralkyle en C₇₋₂₀ portant éventuellement des substituants halogéno, alcoxy en C₁₋₃ ou alkyle en C₁₋₄,
(iv) aryle en C₆₋₁₄ éventuellement halogéné,
(v) alcényle en C₂₋₆,
(vi) cycloalkyle en C₃₋₇,
(vii) (alcoxy en C₁₋₃)-carbonyle,
(viii) mono- ou di-(alkyle en C₁₋₆)-amino,
(ix) (alkyle en C₁₋₆)-carbonyle,
(x) (cycloalkyle en C₃₋₆)oxycarbonyle, ou
(xi) trifluorosulfonyle,
(6) un groupe de formule -S(O)_{f}-R⁶ où f représente un nombre entier compris entre 0 et 2, et R⁶ représente un atome d'hydrogène ou un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement un ou plusieurs substituants choisis dans le groupe formé par halogéno, nitro, cyano, hydroxy, oxo, thioxo, carboxyle, cyano-(aryle en C₆₋₁₄) et halogéno-(aryle en C₆₋₁₄),
(7) un groupe de formule -NR⁹R¹⁰ où R⁹ et R¹⁰ représentent chacun un atome d'hydrogène, un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, acyle en C₁₋₆ ou hétérocyclique à 5 -13 chaînons contenant dans son cycle de 1 à 4 hétéroatomes choisis parmi O, S et N,
(8) un groupe de formule : -COR¹¹ où R¹¹ représente un atome d'hydrogène, un groupe hydroxy, alkyle en C₁₋₆, alcoxy en C₁₋₆, cycloalkyle en C₃₋₆, aryle en C₆₋₁₄, aralkyle en C₇₋₂₀, un groupe de formule -NR⁹R¹⁰ tel que défini ci-dessus, ou un groupe hétérocyclique à 5 -13 chaînons contenant, dans son cycle, de 1 à 4 hétéroatomes choisis parmi O, S et N, éventuellement substitué par des groupes halogéno, alkyle en C₁₋₄, alcoxy en C₁₋₃, alkylthio en C₁₋₄ ou phénoxy éventuellement halogéné,
(9) un groupe hétérocyclique à 5 -13 chaînons contenant, dans son cycle, de 1 à 4 hétéroatomes choisis parmi O, S et N, qui peut porter un ou plusieurs substituants halogéno, alkyle en C₁₋₄, alcoxy en C₁₋₃, alkylthio en C₁₋₄, ou phénoxy éventuellement halogéné,
(10) sulfo,
(11) aryle en C₆₋₁₄,
(12) cycloalkyle en C₃₋₇,
(13) alkylènedioxy en C₁₋₆,
(14) oxo,
(15) thioxo,
(16) alcényle en C₂₋₄,
(17) alcynyle en C₃₋₄,
(18) cycloalkyle en C₃₋₁₀,
(19) alcényle en C₂₋₁₀,
(20) aralkyle en C₇₋₂₀,
(21) amidino et
(22) azido.

7. Composé selon la revendication 1, dans lequel R² représente un groupe alkyle en C₁₋₁₀, cycloalkyle en C₃₋₁₀, bicycloalkyle en C₇₋₁₀, alcényle en C₂₋₁₀, aryle en C₆₋₁₄, aralkyle en C₇₋₂₀, portant éventuellement de 1 à 6 substituants choisis dans le groupe formé par les radicaux (1) halogéno, (2) nitro, (3) hydroxy, (4) cyano, (5) alkylthio en C₁₋₄, (6) alcoxy en C₁₋₆, (7) (alkyle en C₁₋₆)-carbonyloxy ou (8) (cycloalkyle en C₃₋₆)-oxycarbonyle.

8. Composé selon la revendication 1, dans lequel R² représente un atome d'hydrogène ou un groupe alkyle en C₁₋₆ portant éventuellement un ou plusieurs substituants (alkyle en C₁₋₆)-carbonyloxy ou (cycloalkyle en C₃₋₆)-oxycarbonyloxy.

9. Composé selon la revendication 1, dans lequel R³ représente un groupe alkyle en C₁₋₆ portant éventuellement un substituant (alcoxy en C₁₋₆)carbonyle ou un groupe de formule -NH-SO₂-R^{5'} où R^{5'} représente un groupe alkyle en C₁₋₆ ou aryle en C₆₋₁₄.

10. Composé selon la revendication 1, dans lequel R³ représente un groupe alkyle en C₁₋₆, portant un substituant de formule -NH-SO₂-R⁵ où R⁵ représente (1) un groupe alkyle en C₁₋₆ éventuellement halogéné ou (2) un groupe aryle en C₆₋₁₄.

11. Composé selon la revendication 1, dans lequel R³ représente un groupe alkyle en C₁₋₆ portant éventuellement un substituant de formule -NH-SO₂-R^{5'} où R^{5'} est un groupe alkyle en C₁₋₆ ou aryle en C₆₋₁₄.

12. Composé selon la revendication 1, dans lequel
R¹ représente un groupe benzyle portant éventuellement un ou plusieurs substituants (1) halogéno ou (2) alkylthio en C₁₋₄,
R² représente un atome d'hydrogène ou un groupe alkyle en C₁₋₄, portant éventuellement un ou plusieurs substituants (1) (alkyle en C₁₋₄)-carbonyloxy ou (2) (cycloalkyle en C₃₋₆)-oxycarbonyloxy,
R² représente un groupe alkyle en C₁₋₆ portant (1) un groupe (alcoxy en C₁₋₆)-carbonyle ou (2) un groupe de formule : -NH-SO₂-R^{5"} (où R^{5"} est (1) un groupe alkyle en C₁₋₃ éventuellement halogéné ou (2) un groupe phényle,
R⁴ représente un groupe phényle portant éventuellement un ou plusieurs substituants (1) alcoxy en C₁₋₄, à son tour éventuellement substitué par des groupes alcoxy en C₁₋₆, carboxyle, (alkyle en C₁₋₆)-carbamoyle, pipérazinecarbonyle ou halogéno, (2) aralkyloxy en C₇₋₈, (3) alkyle en C₁₋₄ portant éventuellement un substituant alcoxy en C₁₋₃, (4) alcanoyle en C₁₋₆, (5) alcényloxy en C₂₋₄, (6) (alcoxy en C₁₋₆)-carbonyle ou (7) (alkyle en C₁₋₆)-carbamoyle.

13. Acide 2,4-(1H,3H)-dioxo-6-(4-méthoxyméthoxyphényl)-1-(2-méthylthiobenzyl)-5-(méthanesulfonamidométhyl)-thiéno[2,3-d]pyrimidine-3-acétique ou le sel correspondant.

14. Acide 2,4-(1H,3H)-dioxo-6-(4-méthoxyméthoxyphényl)-1-(2-méthylthiobenzyl)-5-(éthanesulfonamidométhyl)-thiéno[2,3-d]pyrimidine-3-acétique ou le sel correspondant.

15. Acide 2,4-(1H,3H)-dioxo-6-(4-méthoxyphényl)-1-(2-méthylthiobenzyl)-5-(méthanesulfonamidométhyl)-thiéno[2,3-d]pyrimidine-3-acétique ou le sel correspondant.

16. 2,4-(1H,3H)-dioxo-6-(4-méthoxyphényl)-1-(2-méthylthiobenzyl)-5-(carboxyméthyl)-thiéno [2,3-d]pyrimidine-3-acétate d'éthyle.

17. Procédé de préparation d'un composé tel que défini dans la revendication 1, comprenant le fait de soumettre un composé de formule 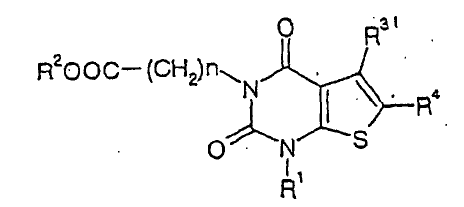 où R^{3'} représente un groupe alkyle en C₁₋₆ halogéné ou portant un groupe cyano, et les autres symboles ont la même signification que dans la revendication 1,
soit (1), lorsque R^{3'} représente un groupe alkyle en C₁₋₆ halogéné, à une réaction de substitution nucléophile avec un sulfonamide,
soit (2), lorsque R^{3'} représente un groupe alkyle en C₁₋₆ portant un groupe cyano, à une réaction d'hydrolyse en milieu alcalin et une estérification.

18. Composition pharmaceutique comprenant un composé tel que défini dans la revendication 1 ou 16 et un support, excipient ou diluant.

19. Composition pharmaceutique selon la revendication 18, qui est un médicament thérapeutique destiné à traiter la vasoconstriction chez un mammifère.

20. Composition pharmaceutique selon la revendication 19, dans laquelle la vasoconstriction est celle des artères coronaires, des veines coronaires, du système cérébrovasculaire ou du système vasculaire pulmonaire.

21. Composition pharmaceutique selon la revendication 18, qui est un médicament ayant une activité antagoniste de l'endothéline.

22. Composition pharmaceutique selon la revendication 21, qui est un médicament therapeutique destiné à traiter l'insuffisance rénale aigüe, l'infarctus du myocarde ou l'insuffisance hépatique.

23. Composition pharmaceutique selon la revendication 21, qui est un médicament therapeutique destiné à traiter l'hypofonctionnement d'un organe dû à une intervention chirurgicale ou une transplantation.

24. Composition pharmaceutique selon la revendication 23, où l'organe est le foie.

25. Utilisation d'un composé tel que défini dans la revendication 1 ou 16, pour la préparation d'une composition pharmaceutique servant à la fabrication d'un médicament destiné au traitement de la vasoconstriction.

26. Utilisation d'un composé tel que défini dans la revendication 1 ou 16, pour la préparation d'une composition pharmaceutique servant à la fabrication d'un médicament destiné au traitement de l'insuffisance rénale aigüe, de l'infarctus du myocarde ou de l'insuffisance hépatique.
